# NEUE EUROPÄISCHE PATENTSCHRIFT

(11) **EP 1 713 760 B2**
(45) Veröffentlichungstag und Bekanntmachung des Hinweises auf die Entscheidung über den Einspruch: **20.03.2019**
(45) Hinweis auf die Patenterteilung: 02.07.2008
(21) Anmeldenummer: 05701204.9
(22) Anmeldetag: 27.01.2005
(51) Int. Cl.: C07C 253/10, C07C 255/04

(54) **VERFAHREN ZUR HERSTELLUNG VON DINITRILEN**
METHOD FOR PRODUCING DINITRILES
PROCEDE POUR PRODUIRE DES DINITRILES

(30) Priorität: 29.01.2004 DE 102004004683
(43) Veröffentlichungstag der Anmeldung: 25.10.2006
(73) Patentinhaber: BASF SE, 67056 Ludwigshafen am Rhein (DE)
(72) Erfinder: JUNGKAMP, Tim, B-2950 Kapellen (BE); BAUMANN, Robert, 68159 Mannheim (DE); BARTSCH, Michael, 67433 Neustadt (DE); HADERLEIN, Gerd, 67269 Grünstadt (DE); LUYKEN, Hermann, 67069 Ludwigshafen (DE); SCHEIDEL, Jens, 69493 Hirschberg (DE)
(74) Vertreter: Féaux de Lacroix, Stefan
(86) Internationale Anmeldenummer: PCT/EP2005/000777
(87) Internationale Veröffentlichungsnummer: WO 2005/073172

(56) Entgegenhaltungen:
- EP-A- 0 464 691
- US-A- 3 564 040
- US-A- 3 773 809
- US-A- 4 080 374
- US-A- 4 385 007

## Beschreibung

Die vorliegende Erfindung betrifft ein Verfahren zur Herstellung von Adipodinitril und Methylglutarnitril.

Eine Beschreibung der Grundzüge eines zusammenhängenden Verfahrens zur Herstellung von Adipodinitril kann US 4,080,374 entnommen werden, wobei 1,3-Butadien beispielsweise gemäß US 3,496,215 mit Cyanwasserstoff unter Erhalt einer Mischung, enthaltend 3-Pentennitril und 2-Methyl-3-butennitril, umgesetzt wird. 2-Methyl-3-butennitril kann beispielsweise gemäß US 3,542,874 zu 3-Pentennitril isomerisiert werden. In einem weiteren Schritt wird das so erhaltene 3-Pentennitril beispielsweise gemäß US3,752,839 mit Cyanwasserstoff zu einer Mischung umgesetzt, enthaltend Adipodinitril und Methylglutarnitril.

Die bekannten Verfahren zur Herstellung von Adipodinitril und Methylglutarnitril weisen eine Vielzahl von Verfahrensschritten auf. Auch einzelne Verfahrenschritte und Ausgestaltungen und deren Verbesserungen sind bereits diskutiert worden.

So beschreiben beispielsweise US 3,773,809 und US 4,385,007 Verfahren zur Extraktion von Nickel(0)-Katalysatoren, die in Hydrocyanierungsprozessen verwendet werden, aus Mischungen, die 3- bzw. 4-Pentennitrile enthalten. Dabei wird ein Umsatz von mehr als 60 % in der Hydrocyanierung vorausgesetzt. Diese Umsatzgrade lassen sich jedoch nur mit großen Verlusten an Nickel(0)-Katalysatoren erreichen.

Die Behandlung der in der Extraktion erhaltenen Ströme ist in US 4,080,374 beschrieben, welche ein Verfahren zur Aufarbeitung von Hydrocyanierungsausträgen durch Extraktion, Zentrifugieren des Raffinats und anschließender Destillation beschreibt, um Nitrile und das Extraktionsmittel aus der Adipodinitril-reichen Phase der Extraktion zurückzugewinnen. Bei dieser Rückgewinnung werden jedoch durch die Notwendigkeit, das Extraktionsmittel und Pentennitril aus dem Katalysatorstrom möglichst vollständig zurückzugewinnen, bei technisch sinnvollen Drücken so hohe Temperaturen erreicht, die zu einer zumindest partiellen Zersetzung des Wertproduktes Adipodinitril, beispielsweise durch Reaktion mit dem jeweilig verwendeten Promotor Zinkchlorid oder Triphenylbor führen.

Bezüglich der Aufarbeitung von Reaktionsausträgen der Hydrocyanierung von Pentennitril zu Adipodinitril mit Rückführung von nicht umgesetztem Pentennitril ist ferner US 3,564,040 zu erwähnen, welche die Entfernung von trans-2-Pentennitril aus einer Mischung aus cis-, trans-3-Pentennitrilen und 4-Pentennitrilen durch katalytische Isomerisierung des trans-2-Pentennitrils zu cis-2-Pentennitril beschreibt. An die Isomerisierung schließt sich eine fraktionierende Destillation zur Abtrennung von cis-2-Pentennitril und eine Rückführung des Pentennitrilstromes mit dem restlichen trans-2-Pentennitril, welches im Reaktor zu cis-2-Pentennitril isomerisiert wird, an. Aus den Beispielen der US 3,564,040 ergibt sich, dass eine Vergiftung der Katalysatoren für die Hydrocyanierung bereits durch 2,5 Gew.- % trans-2-Pentennitril gegeben ist.

Bezüglich der Abtrennung von cis-2-Pentennitrilen aus Mischungen von 3- und 4-Pentennitrilen sind US 3,852,325 und US 3,852,327 zu erwähnen, welche die Isomerisierung von trans-2-Pentennitril mit Triarylborhalogenid bzw. Nickel(0) mit Tritolylphosphit-Liganden mit Lewissäurezusatz beschreiben, um trans-2-Pentennitril in cis-2-Pentennitril zu überführen, das sich leichter von 3-Pentennitrilen abtrennen lässt.

Die Aufarbeitung von Adipodinitril ist in US 3,766,241 beschrieben, welche die Aufarbeitung von Hydrocyanierungsausträgen durch deren Behandlung mit Ammoniak betrifft, um Zink(II)-chlorid, das in der Hydrocyanierung als Promotor verwendet wird, als Ammoniak-Addukt auszufällen.

Die bisher bekannten einzelnen Verfahrensschritte, die nötig sind, um aus einem Hydrocyanierungsgemisch Adipodinitril zu isolieren sowie nicht umgesetztes Pentennitril und den Nickel(0)-Katalysator in das Verfahren zur Vermeidung von Einsatzstoffverlusten zurückzuführen, führen durch Wertprodukt- und Katalysatorverluste nicht zu zufrieden stellenden Ergebnissen.

Demnach war es Aufgabe der vorliegenden Erfindung, ein integriertes Verfahren zur Herstellung von Adipodinitril und Methylglutarnitril bereitzustellen, das die zuvor diskutierten Nachteile der Verfahren des Standes der Technik im Wesentlichen vermeidet und vorzugsweise die nachstehend aufgeführten Vorteile zeigt.

Diese Aufgabe wird gelöst durch das erfindungsgemäße Verfahren zur Herstellung von Adipodinitril und Methylglutarnitril.

Das erfindungsgemäße Verfahren ist durch die folgenden Verfahrensschritte gekennzeichnet:
(a) Umsetzung eines Pentennitrile enthaltenden Eduktstroms mit Cyanwasserstoff in Gegenwart mindestens eines Katalysators und mindestens eines Promotors unter Erhalt eines Reaktionsstroms, der Pentennitrile, den mindestens einen Nickel(O)Phosphorligand-Komplex als Katalysator, Katalysatorabbauprodukte, den mindestens einen Promotor aus der Gruppe bestehend aus den Lewissäuren ZnCl₂, FeCl₂, Et₂AlCl, Et₃Al₂Cl₃, EtAlCl₂ und BPh₃, Adipodinitril und Methylglutarnitril, enthält,
(b) Destillation des Reaktionsstroms unter Erhalt eines an Pentennitrilen auf 5 bis 30 Gew.-% abgereicherten Stromes 3, der den mindestens einen Katalysator, Katalysatorabbauprodukte, den mindestens einen Promotor, Adipodinitril und Methylglutarnitril enthält, als Sumpfprodukt und eines an Pentennitrilen angereicherten Stromes 4 als Kopfprodukt,
(c) Extraktion des Stromes 3 mit einem Extraktionsmittel, das ausgewählt ist aus der Gruppe, bestehend aus Cyclohexan, Methylcyclohexan, n-Hexan, n-Heptan, isomeren C6-, C7-, C8-, C9-Cycloaliphaten, isomeren C6-, C7-, C8-, C9-Isoaliphaten, cis-, trans-Decahydronaphthalin und Gemischen davon enthalten in Strom 5 unter Erhalt eines mit Extraktionsmittel angereicherten Stromes 6 als Kopfprodukt, der den Katalysator enthält, und eines an Extraktionsmittel abgereicherten Stromes 7 als Sumpfprodukt, der Katalysatorabbauprodukte, den mindestens einen Promotor, Pentennitrile, Adipodinitril und Methylglutarnitril enthält,
(d) Destillation des Stromes 6 unter Erhalt eines den Katalysator enthaltenden Stromes 8 als Sumpfprodukt und eines das Extraktionsmittel enthaltenden Stromes 9 als Kopfprodukt, wobei Strom 9 weniger als 10 Gew.-% Pentennitrile enthält und wobei 3-Pentennitril als Zwischensieder zur Destillation gegeben wird,
(e) Destillation des Stromes 7 unter Erhalt eines Stromes 10 als Sumpfprodukt, der Katalysatorabbauprodukte, den mindestens einen Promotor, Pentennitrile, Adipodinitril und Methylglutarnitril enthält, und eines das Extraktionsmittel enthaltenden Stromes 11 als Kopfprodukt,
(f) Destillation des Stromes 10 unter Erhalt eines Stromes 12 als Sumpfprodukt, der Katalysatorabbauprodukte, den mindestens einen Promotor, Adipodinitril und Methylglutarnitril enthält, und eines Pentennitrile enthaltenden Stromes 13 als Kopfprodukt, wobei der an Pentennitrilen angereicherte Strom 4 und/oder der Strom 13 cis-2-Pentennitril und (E)-2-Methyl-2-butennitril enthält und zumindest teilweise unter Erhalt eines an cis-2-Pentennitril und (E)-2-Methyl-2-butennitril abgereicherten Stromes 18 und eines an cis-2-Pentennitril und (E)-2-Methyl-2-butennitril angereicherten Stromes 19 destilliert und der Strom 18 zumindest teilweise in Verfahrensschritt (a) zurückgeführt wird.
(g) Destillation des Stromes 12 unter Erhalt eines Stromes 14 als Sumpfprodukt, der Katalysatorabbauprodukte und den mindestens einen Promotor enthält, und eines Stromes 15 als Kopfprodukt, der Adipodinitril und Methylglutarnitril enthält,
(h) Destillation des Stromes 15 unter Erhalt eines Adipodinitril enthaltenden Stromes 16 als Sumpf und eines Methylglutarnitril enthaltenden Stromes 17 als Kopfprodukt.

In *Verfahrensstufe (a)* erfolgt die Umsetzung eines Eduktstromes, der Pentennitrile enthält, mit Cyanwasserstoff in Gegenwart mindestens eines Katalysators und mindestens eines Promotors unter Erhalt eines Reaktionsstroms, der Pentennitrile, den mindestens einen Katalysator, Katalysatorabbauprodukte, den mindestens einen Promotor, Adipodinitril und Methylglutarnitril enthält. Weiterer Bestandteil des Reaktionsstroms ist Valeriansäure.

Der hierbei eingesetzte Eduktstrom stammt vorzugsweise aus der homogenen Hydrocyanierung von Butadien in Gegenwart eines Nickel(0)-Katalysators, die aus dem Stand der Technik an sich bekannt ist.

Als Katalysator in Verfahrensschritt (a) werden Nickel(0)-Phosphortigand-Komptexe verwendet. Bei den Ni(0)-Komplexen, die phosphorhaltige Liganden und/oder freie phosphorhaltige Liganden enthalten, handelt es sich bevorzugt um homogen gelöste Nickel(0)-Komplexe.

Die phosphorhaltigen Liganden der Nickel(0)-Komplexe und die freien phosphorhaltigen Liganden sind vorzugsweise ausgewählt aus mono- oder bidentaten Phosphinen, Phosphiten, Phosphiniten und Phosphoniten.

Diese phosphorhaltigen Liganden weisen vorzugsweise die Formel I auf:

P(X¹R¹) (X²R²) (X³R³) (I)

Unter Verbindung I wird im Sinne der vorliegenden Erfindung eine einzelne Verbindung oder ein Gemisch verschiedener Verbindungen der vorgenannten Formel verstanden.

Erfindungsgemäß sind X¹, X², X³ unabhängig voneinander Sauerstoff oder Einzelbindung. Falls alle der Gruppen X¹, X² und X³ für Einzelbindungen stehen, so stellt Verbindung I ein Phosphin der Formel P(R¹R²R³) mit den für R¹, R² und R³ in dieser Beschreibung genannten Bedeutungen dar.

Falls zwei der Gruppen X¹, X² und X³ für Einzelbindungen stehen und eine für Sauerstoff, so stellt Verbindung I ein Phosphinit der Formel P(OR¹)(R²)(R³) oder P(R¹)(OR²)(R³) oder P(R¹)(R²)(OR³) mit den für R¹, R² und R³ weiter unten genannten Bedeutungen dar.

Falls eine der Gruppen X¹, X² und X³ für eine Einzelbindung steht und zwei für Sauer stoff, so stellt Verbindung I ein Phosphonit der Formel P(OR¹)(OR²)(R³) oder P(R¹)(OR²)(OR³) oder P(OR¹)(R²)(OR³) mit den für R¹, R² und R³ in dieser Beschreibung genannten Bedeutungen dar.

In einer bevorzugten Ausführungsform sollten alle der Gruppen X¹, X² und X³ für Sauerstoff stehen, so dass Verbindung I vorteilhaft ein Phosphit der Formel P(OR¹)(OR²)(OR³) mit den für R¹, R² und R³ weiter unten genannten Bedeutungen darstellt.

Erfindungsgemäß stehen R¹, R², R³ unabhängig voneinander für gleiche oder unterschiedliche organische Reste. Als R¹, R² und R³ kommen unabhängig voneinander Alkylreste, vorzugsweise mit 1 bis 10 Kohlenstoffatomen, wie Methyl, Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorzugsweise mit 1 bis 20 Kohlenstoffatomen, wie 1,1'-Biphenol, 1,1'-Binaphthol in Betracht. Die Gruppen R¹, R² und R³ können miteinander direkt, also nicht allein über das zentrale Phosphor-Atom, verbunden sein. Vorzugsweise sind die Gruppen R¹, R² und R³ nicht miteinander direkt verbunden.

In einer bevorzugten Ausführungsform kommen als Gruppen R¹, R² und R³ Reste ausgewählt aus der Gruppe bestehend aus Phenyl, o-Tolyl, m-Tolyl und p-Tolyl in Betracht. In einer besonders bevorzugten Ausführungsform sollten dabei maximal zwei der Gruppen R¹, R² und R³ Phenyl-Gruppen sein.

In einer anderen bevorzugten Ausführungsform sollten dabei maximal zwei der Gruppen R¹, R² und R³ o-Tolyl-Gruppen sein.

Als besonders bevorzugte Verbindungen I können solche der Formel Ia

(o-Tolyl-O-)_{w} (m-Tolyl-O-)ₓ (p-Tolyl-O-)_{y} (Phenyl-O-)_{z} P (Ia)

eingesetzt werden, wobei w, x, y und z eine natürliche Zahl bedeuten, und folgende Bedingungen gelten: w + x + y + z = 3 und w, z ≤ 2.

Solche Verbindungen I a sind z.B. (p-Tolyl-O-)(Phenyl-O-)₂P, (m-Tolyl-O-)(Phenyl-O-)₂P, (o-Tolyl-O-) (Phenyl-O-)₂P, (p-Tolyl-O-)₂(Phenyl-O-)P, (m-Tolyl-O-)₂(Phenyl-O-)P, (o-Tolyl-O-)₂(Phenyl-O-)P, (m-Tolyl-O-)(p-Tolyl-O)(Phenyl-O-)P, (o-Tolyl-O-)(p-Tolyl-O-)(Phenyl-O-)P, (o-Tolyl-O-)(m-Tolyl-O-)(Phenyl-O-)P, (p-Tolyl-O-)₃P, (m-Tolyl-O-)(p-Tolyl-O-)₂P, (o-Tolyl-O-)(p-Tolyl-O-)₂P, (m-Tolyl-O-)₂(p-Toluyl-O-)P, (o-Tolyl-O-)₂(p-Tolyl-O-)P, (o-Tolyl-O-)(m-Tolyl-O-)(p-Tolyl-O)P, (m-Tolyl-O-)₃P, (o-Tolyl-O-)(m-Tolyl-O-)₂P (o-Tolyl-O-)₂(m-Tolyl-O-)P, oder Gemische solcher Verbindungen.

Gemische enthaltend (m-Tolyl-O-)₃P, (m -Tolyl-O-)₂(p-Tolyl-O-)P, (m-Tolyl-O-)(p-Tolyl-O-)₂P und (p-Tolyl-O-)₃P kann man beispielsweise durch Umsetzung eines Gemisches enthaltend m-Kresol und p-Kresol, insbesondere im Molverhältnis 2: 1, wie es bei der destillativen Aufarbeitung von Erdöl anfällt, mit einem Phosphortrihalogenid, wie Phosphortrichlorid, erhalten.

In einer anderen, ebenfalls bevorzugten Ausführungsform kommen als phosphorhaltige Liganden die in der DE-A 199 53 058 näher beschriebenen Phosphite der Formel I b in Betracht:

P (O-R¹)ₓ (O-R²)_{y} (O-R³)_{z} (O-R⁴)ₚ (Ib)

mit
- R¹:: aromatischer Rest mit einem C₁-C₁₈-Alkylsubstituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromatischen System verbindet, anellierten aromatischen System,
- R²:: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der aromatische Rest in o-Stellung zu dem Sauerstoffatom, das das Phosphoratom mit dem aromatischen System verbindet, ein Wasserstoffatom trägt,
- R³:: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aromatische Rest in o-Stellung zu dem Sauerstoffatom, das das Phosphoratom mit dem aromatischen System verbindet, ein Wasserstoffatom trägt,
- R⁴:: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt,
- x:: 1 oder 2,
- y, z, p:: unabhängig voneinander 0, 1 oder 2 mit der Maßgabe, dass x+y+z+p = 3.

Bevorzugte Phosphite der Formel Ib sind der DE-A 199 53 058 zu entnehmen. Als Rest R¹ kommen vorteilhaft o-Tolyl-, o-Ethyl-phenyl-, o-n-Propyl-phenyl-, o-Isopropyl-phenyl-, o-n-Butyl-phenyl-, o-sek-Butyl-phenyl-, o-tert-Butyl-phenyl-, (o-Phenyl)-Phenyl-oder 1-Naphthyl- Gruppen in Betracht.

Als Rest R² sind m-Tolyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen bevorzugt.

Als Rest R³ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Rest R⁴ ist bevorzugt Phenyl. Vorzugsweise ist p gleich null. Für die Indizes x, y, z und p in Verbindung Ib ergeben sich folgende Möglichkeiten:

| x | y | z | p |
|---|---|---|---|
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

Bevorzugte Phosphite der Formel I b sind solche, in denen p gleich null ist sowie R¹, R² und R³ unabhängig voneinander ausgewählt sind aus o-Isopropyl-phenyl, m-Tolyl und p-Tolyl, und R⁴ Phenyl ist.

Besonders bevorzugte Phosphite der Formel I b sind solche, in denen R¹ der o-Isopropyl-phenyl-Rest, R² der m-Tolylrest und R³ der p-Tolylrest ist mit den in der vorstehenden Tabelle genannten Indizes; außerdem solche, in denen R¹ der o-Tolylrest, R² der m-Tolylrest und R³ der p-Tolylrest ist mit den in der Tabelle genannten Indizes; weiterhin solche, in denen R¹ der 1-Naphthylrest, R² der m-Tolylrest und R³ der p-Tolylrest ist mit den in der Tabelle genannten Indizes; außerdem solche, in denen R¹ der o-Tolylrest, R² der 2-Naphthylrest und R³ der p-Tolylrest ist mit den in der Tabelle genannten Indizes; und schließlich solche, in denen R¹ der o-Isopropyl-phenyl-Rest, R² der 2-Naphthylrest und R³ der p-Tolylrest ist mit den in der Tabelle genannten Indizes; sowie Gemische dieser Phosphite.

Phosphite der Formel Ib können erhalten werden, indem man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹OH, R²OH, R³OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹OH, R²OH, R³OH und R⁴OH oder deren Gemische umsetzt unter Erhalt eines Phosphits der Formel I b.

Die Umsetzung kann in drei getrennten Schritten durchgeführt werden. Ebenso können zwei der drei Schritte kombiniert werden, also a) mit b) oder b) mit c). Alternativ können alle der Schritte a), b) und c) miteinander kombiniert werden.

Dabei kann man geeignete Parameter und Mengen der Alkohole ausgewählt aus der Gruppe bestehend aus R¹OH, R²OH, R³OH und R⁴OH oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere Cl, eingesetzt wird, sowie deren Gemische in Betracht. Es können auch Gemische verschiedener gleich oder unterschiedlich halogensubstituierter Phosphine als Phosphortrihalogenid eingesetzt werden. Besonders bevorzugt ist PCl₃. Weitere Einzelheiten zu den Reaktionsbedingungen bei der Herstellung der Phosphite Ib und zur Aufarbeitung sind der DE-A 199 53 058 zu entnehmen.

Die Phosphite I b können auch in Form eines Gemisches verschiedener Phosphite Ib als Ligand verwendet werden. Ein solches Gemisch kann beispielsweise bei der Herstellung der Phosphite I b anfallen.

Es ist allerdings bevorzugt, dass der phosphorhaltige Ligand mehrzähnig, insbesondere zweizähnig ist. Daher weist der verwendete Ligand vorzugsweise die Formel II 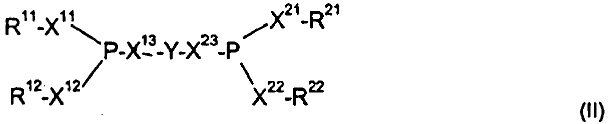 auf, worin bedeuten
- X¹¹, X¹², X¹³, X²¹, X²², X²³: unabhängig voneinander Sauerstoff oder Einzelbindung
- R¹¹, R¹²: unabhängig voneinander gleiche oder unterschiedliche, einzelne oder verbrückte organische Reste
- R²¹, R²²: unabhängig voneinander gleiche oder unterschiedliche, einzelne oder verbrückte organische Reste,
- Y: Brückengruppe

Unter Verbindung II wird im Sinne der vorliegenden Erfindung eine einzelne Verbindung oder ein Gemisch verschiedener Verbindungen der vorgenannten Formel verstanden.

In einer bevorzugten Ausführungsform können X¹¹, X¹², X¹³, X²¹, X²², X²³ Sauerstoff darstellen. In einem solchen Fall ist die Brückengruppe Y mit Phosphit-Gruppen verknüpft.

In einer anderen bevorzugten Ausführungsform können X¹¹ und X¹² Sauerstoff und X¹³ eine Einzelbindung oder X¹¹ und X¹³ Sauerstoff und X¹² eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen bevorzugten Ausführungsform können X¹³ Sauerstoff und X¹¹ und X¹² eine Einzelbindung oder X¹¹ Sauerstoff und X¹² und X¹³ eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphinits oder Phosphins, vorzugsweise eines Phosphinits, sein kann.

In einer anderen bevorzugten Ausführungsform können X¹¹, X¹² und X¹³ eine Einzelbindung darstellen, so dass das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so dass das mit X²¹, X²² und X²³ umgebene Phosphoratom Zentralatom eines Phosphits oder Phosphins, vorzugsweise eines Phosphins, sein kann.

Als Brückengruppe Y kommen vorzugsweise substituierte, beispielsweise mit C₁-C₄-Alkyl, Halogen, wie Fluor, Chlor, Brom, halogeniertem Alkyl, wie Trifluormethyl, Aryl, wie Phenyl, oder unsubstituerte Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterscheidliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R¹¹ und R¹² können einzeln oder verbrückt sein. Auch die Reste R²¹ und R²² können einzeln oder verbrückt sein. Die Reste R¹¹, R¹², R²¹ und R²² können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht. In einer besonders bevorzugten Ausführungsform kommen die in US 5,512,696 genannten Verbindungen der Formel I, II, III IV, V, VI und VII, insbesondere die dort in den Beispielen 1 bis 31 eingesetzten Verbindungen, in Betracht. In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzten Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzten Verbindungen, in Betracht. In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzten Verbindungen, in Betracht.

In einer besonders bevorzugten Ausführungsform kommen die in US 6,127,567 genannten Verbindungen und dort in den Beispielen 1 bis 29 eingesetzten Verbindungen in Betracht. In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzten Verbindungen, in Betracht. In einer besonders bevorzugten Ausführungsform kommen die in US 5,959,135 genannten Verbindungen und dort in den Beispielen 1 bis 13 eingesetzten Verbindungen in Betracht.

In einer besonders bevorzugten Ausführungsform kommen die in US 5,847,191 genannten Verbindungen der Formel I, II und III in Betracht. In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 13, 14, 15, 16, 17, 18, 19, 20 und 21 dargestellten Verbindungen, in Betracht. In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht. In einer besonders bevorzugten Ausführungsform kommen die in WO 99/13983 genannten Verbindungen in Betracht. In einer besonders bevorzugten Ausführungsform kommen die in WO 99/64155 genannten Verbindungen in Betracht.

In einer besonders bevorzugten Ausführungsform kommen die in der deutschen Patentanmeldung DE 100 380 37 genannten Verbindungen in Betracht. In einer besonders bevorzugten Ausführungsform kommen die in der deutschen Patentanmeldung DE 100 460 25 genannten Verbindungen in Betracht. In einer besonders bevorzugten Ausführungsform kommen die in der deutschen Patentanmeldung DE 101 502 85 genannten Verbindungen in Betracht.

In einer besonders bevorzugten Ausführungsform kommen die in der deutschen Patentanmeldung DE 101 502 86 genannten Verbindungen in Betracht. In einer besonders bevorzugten Ausführungsform kommen die in der deutschen Patentanmeldung DE 102 071 65 genannten Verbindungen in Betracht. In einer weiteren besonders bevorzugten Ausführungsform der vorliegenden Erfindung kommen die in der US 2003/0100442 A1 genannten phosphorhaltigen Chelatliganden in Betracht.

In einer weiteren besonders bevorzugten Ausführungsform der vorliegenden Erfindung kommen die in der nicht vorveröffentlichten deutschen Patentanmeldung Aktenzeichen DE 103 50 999:2 vom 30.10.2003 genannten phosphorhaltigen Chelatliganden in Betracht.

Die beschriebenen Verbindungen I, Ia, Ib und II sowie deren Herstellung sind an sich bekannt. Als phosphorhaltiger Ligand können auch Mischungen, enthaltend mindestens zwei der Verbindungen I, Ia, Ib und II, eingesetzt werden.

In einer besonders bevorzugten Ausführungsform des erfindungsgemäßen Verfahrens ist der phosphorhaltige Ligand des Nickel(0)-Komplexes und/oder der freie phosphorhaltige Ligand ausgewählt aus Tritolylphosphit, bidentaten phosphorhaltigen Chelatliganden, sowie den Phosphiten der Formel I b

P (O-R¹)ₓ (O-R²)_{y} (O-R³)_{z} (O-R⁴)ₚ (Ib)

worin R¹, R² und R³ unabhängig voneinander ausgewählt sind aus o-Isopropyl-phenyl, m-Tolyl und p-Tolyl, R⁴ Phenyl ist; x gleich 1 oder 2 ist, und y, z, p unabhängig voneinander 0, 1 oder 2 sind mit der Maßgabe, dass x+y+z+p = 3 ist; und deren Mischungen.

Der Verfahrensschritt (a) des erfindungsgemäßen Verfahrens wird vorzugsweise bei einem absoluten Druck von 0,1 bis 10 bar, besonders bevorzugt 0,5 bis 2 bar, insbesondere 0,8 bis 1,5 bar, durchgeführt. Die Temperatur beträgt in Verfahrensschritt (a) vorzugsweise 40 bis 150 °C, besonders bevorzugt 50 bis 100 °C, insbesondere 60 bis 70 °C.

Der Verfahrensschritt (a) kann in jeder geeigneten, dem Fachmann bekannten Apparatur durchgeführt werden. Geeignet sind beispielsweise Reaktoren mit Rückvermischungscharakteristik.

In der Hydrocyanierung des Verfahrensschrittes (a) kann es zu einer partiellen Zersetzung des Katalysators kommen. Die hieraus resultierenden Abbauprodukte sind beispielsweise Nickel(II)-cyanid-haltige Komponenten. Als weitere Abbauprodukte entstehen beispielsweise hydrolysierte Phosphorverbindungen, die sich durch Reaktion der jeweilig eingesetzten Stoffe **I** und **II** durch Spuren von Wasser ableiten, die in den Einsatzstoffen enthalten sein können, insbesondere im eingesetzten Cyanwasserstoff. Als weitere Abbauprodukte entstehen oxidierte Phosphorverbindungen, die sich durch Reaktion der jeweilig eingesetzten Stoffe **I** und **II** zu Verbindungen mit Phosphoratomen in der Oxidationsstufe (V) durch Reaktion mit elementarem Sauerstoff oder durch Reaktion mit Peroxiden ableiten, die in den Einsatzstoffen enthalten sein können, insbesondere durch Leckage in der Apparatur oder durch Einlösen von Sauerstoff in Pentennitril, beispielsweise bei Lagerung, unter nachfolgender Bildung von PeroxidVerbindungen der Pentennitrile. Als weitere Abbauprodukte, insbesondere der eingesetzten Chelatliganden **II**, können unter unvorteilhaften Bedingungen monodentate Abbauprodukte, die sich durch beispielsweise thermisch induzierte oder protonen- oder basenkatalysierte Umlagerung der Reste an den Phosphoratomen der jeweiligen Strukturen ableiten und weniger Hydrocyanierungsaktivität als die Ausgangsstoffe besitzen, entstehen.

In der Hydrocyanierung des Verfahrensschrittes (a) bilden sich darüber hinaus im Allgemeinen 2-Pentennitrile, die bei der Verwendung von Nickel(0)-Katalysatoren mit monodentaten Liganden mit ZnCl₂ oder BPh₃ als Promotor Katalysatorgifte sind.

Der Promotor, der in Verfahrensschritt (a) des erfindungsgemäßen Verfahrens eingesetzt wird, wird ausgewählt aus der Gruppe bestehend aus den Lewissäuren ZnCl₂, FeCl₂, Et₂AlCl, Et₃Al₂Cl₃, EtAlCl₂ und BPh₃.

Der Verfahrensschritt (a) kann in jeder geeigneten, dem Fachmann bekannten Vorrichtung durchgeführt werden. Für die Reaktion kommen hierfür übliche Apparaturen in Betracht, wie sie beispielsweise in: Kirk-Othmer, Encyclopedia of Chemical Technology, 4. Ed. Vol. 20, John Wiley & Sons, New York 1996, Seiten 1040 bis 1055 beschrieben sind, wie Rührkesselreaktoren, Schlaufenreaktoren, Gasumlaufreaktoren, Blasensäulenreaktoren oder Rohrreaktoren, jeweils gegebenenfalls mit Vorrichtungen zur Abfuhr von Reaktionswärme. Die Reaktion kann in mehreren, wie zwei oder drei, Apparaten durchgeführt werden.

In *Verfahrensschritt (b)* erfolgt eine Destillation des Reaktionsstroms unter Erhalt eines an Pentennitrilen auf 5 bis 30 Gew.-% abgereicherten Stromes 3 als Sumpfprodukt, der den mindestens einen Katalysator, Katalysatorabbauprodukte, den mindestens einen Promotor, Adipodinitril und Methylglutarnitril enthält, und eines an Pentennitrilen angereicherten Stromes 4 als Kopfprodukt.

Eine wesentliche Abtrennung der Pentennitrile vor der sich anschließenden Extraktion in Verfahrensschritt (c) ist vorteilhaft, da andernfalls eine Phasentrennung in der Extraktion gegebenenfalls erschwert oder verhindert wird und die Effizienz der Extraktion von Katalysatorbestandteilen aus dem Hydrocyanierungsaustrag durch hohe Pentennitrilkonzentration nachteilig beeinflusst wird.

Die Verdampfung von Verfahrensschritt (b) kann einstufig oder in mehreren in Serie ausgeführten Stufen bei unterschiedlichen Temperaturen und Drücken erfolgen. Weiterhin kann die Verdampferstufe von Verfahrensschritt(b) als Destillationskolonne ausgeführt werden, wobei der Betrieb als Verstärkungs- oder Abtriebskolonne möglich ist. In einer bevorzugten Ausführungsform wird die Verdampferstufe (b) als Destillationskolonne in Abtriebsfahrweise betrieben.

In einer weiteren bevorzugten Ausführungsform des erfindungsgemäßen Verfahrens wird mindestens eine der Verdampferstufen von Verfahrensschritt (b) mit einem geteilten Sumpf betrieben, wobei man aus einem ersten Sumpf der betreffenden Verdampferstufe den im Verhältnis zum Sumpfabzugsstrom im Allgemeinen großen Umlaufstrom zum Verdampfer fährt, den flüssigen Ablaufstrom aus dem Verdampfer jedoch nicht direkt in den Sumpf zurückgibt, sondern in einen zweiten Sumpf, der vom ersten Sumpf getrennt ist, auffängt, aus dem zweiten Sumpf den Sumpfabzugsstrom erhält und den verbleibenden Überschuss vom Verdampferumlaufstrom in den ersten Sumpf überlaufen lässt, wobei als Sumpfabzugsstrom aus dem zweiten Sumpf eine Mischung erhalten wird, die gegenüber dem Abzug aus dem ersten Sumpf an Leichtsiedern abgereichert ist.

Der absolute Druck in Verfahrenschritt (b) beträgt vorzugsweise 0,001 bis 1 bar, besonders bevorzugt 0,005 bis 0,1 bar, insbesondere 0,01 bis 0,05 bar. Die Destillation wird so durchgeführt, dass die Temperatur im Sumpf der Destillationsapparatur vorzugsweise 40 bis 180 °C, besonders bevorzugt 70 bis 120 °C, insbesondere 80 bis 100 °C, beträgt. Die Destillation wird so durchgeführt, dass die Temperatur am Kopf der Destillationsapparatur vorzugsweise -15 bis 150 °C, besonders bevorzugt 0 bis 60 °C, insbesondere 20 bis 50 °C, beträgt. In einer besonders bevorzugten Ausführungsform des erfindungsgemäßen Verfahrens werden die zuvor genannten Temperaturbereiche sowohl am Kopf als auch im Sumpf eingehalten.

Der in Verfahrensschritt (b) an Pentennitrilen angereicherte Strom 4 enthält im Allgemeinen trans-3-Pentennitril, cis-3-Pentennitril, 4-Pentennitril, cis-2-Pentennitril, trans-2-Pentennitril und (*E*)-2-Methyl-2-butennitril. In einer besonders bevorzugten Ausführungsform wird dieser Strom 4 zumindest teilweise unter Erhalt eines an cis-2-Pentennitril und (E)-2-Methyl-2-butennitril abgereicherten Stromes 18 und eines an cis-2-Pentennitril und (E)-2-Methyl-2-butennitril angereicherten Stromes 19 in einem weiteren Verfahrensschritt (i) destilliert. Der an cis-2-Pentennitril und (E)-2-Methyl-2-butennitril abgereicherten Stromes 18 wird in die Hydrocyanierung des Verfahrensschrittes (a) zurückgeführt Strom 19 kann einer weiteren Aufarbeitung zugeführt werden, um zur Rückgewinnung von Wertprodukt das cis-2-Pentennitril zu 3-Pentennitril zu isomerisieren, beispielsweise gemäß einem Verfahren wie in DE 103 23 803 oder in der DE-A-102 004 004 716 beschrieben.

Die Destillation des Verfahrensschrittes (i) kann in jeder geeigneten, dem Fachmann bekannten Apparatur durchgeführt werden. Die Destillation des Verfahrensschrittes (i) findet vorzugsweise in einer oder mehreren Destillationskolonnen statt. Die Kolonnen können mit einem oder mehreren Seitenabzügen ausgestattet sein. Als Einbauten für die Destillationskolonnen werden vorzugsweise strukturierte Blechpackungen, strukturierte Gewebepackungen, Glockenbödenen, Dual-flow-Böden oder Schüttungen aus Füllkörpern oder Kombinationen von zwei oder mehreren dieser Klassen von trennwirksamen Einbauten verwendet.

Eine oder mehrere Kolonnen des Verfahrensschrittes (i) können als Trennwandkolonne mit Seitenabzug ausgeführt werden. Die Kolonnen des Verfahrensschrittes (i) können mit Fallfilmverdampfer, Dünnschichtverdampfer, Naturumlaufverdampfer oder Zwangsumlaufentspannungsverdampfer als Verdampfereinheit ausgerüstet sein.

Der absolute Druck in Verfahrensschritt (i) beträgt vorzugsweise 0,01 bis 10,0 bar, besonders bevorzugt 0,05 bis 5,0 bar, insbesondere 0,1 bis 1,0 bar. Die Destillation wird so durchgeführt, dass die Temperatur im Sumpf der Destillationsapparatur vorzugsweise 40 bis 250 °C, besonders bevorzugt 50 bis 180 °C. insbesondere 60 bis 150 °C. beträgt. Die Destillation wird so durchgeführt, dass die Temperatur am Kopf der Destillationsapparatur vorzugsweise 0 bis 200 °C, besonders bevorzugt 15 bis 180 °C, insbesondere 20 bis 150 °C, beträgt. In einer besonders bevorzugten Ausführungsform des erfindungsgemäßen Verfahrens werden die zuvor genannten Temperaturbereiche in dem Verfahrensschritt (i) sowohl am Kopf als auch im Sumpf eingehalten.

Erfindungsgemäß wurde festgestellt, dass - im Widerspruch zum Stand der Technik - 2-Pentennitrile keine Katalysatorgifte darstellen, wenn die Hydrocyanierung unter Verwendung von Nickel(0)-Katalysatoren mit Chelatliganden durchgeführt wird. Daher ist es nicht zwingend, dass cis-2-Pentennitril und (E)-2-Methyl-2-butennitril aus dem in die Hydrocyanierung zurückgeführten Strom 18 vollständig entfernt werden, wenn in der Hydrocyanierung des Verfahrensschritts (a) ein Nickel(0)-Katalysator mit Chelatliganden verwendet wird.

Der in Verfahrensschritt (b) an Pentennitril abgereicherte Strom 3 enthält noch 5 bis 30 Gew.-%, Pentennitrile. Diese Pentennitrile setzen sich im Allgemeinen zusammen aus cis-3-Pentennitril, trans-3-Pentennitril und 4-Pentennitril sowie zusätzlich, jeweils mit einem Anteil von vorzugsweise weniger als 20 Gew.-%, besonders bevor zugt weniger als 10 Gew.-%, cis-2-Pentennitril, trans-2-Pentennitril, (E)-2-Methyl-2-butennitril und (Z)-2-Methyl-2-butennitril. Weitere Bestandteile sind in der Hydrocyanierung gebildete Nebenprodukte in untergeordnetem Maße, beispielsweise Valeriansäurenitril.

In *Verfahrensschritt (c)* erfolgt eine Extraktion des Stromes 3 mit einem Extraktionsmittel in Strom 5 unter Erhalt eines mit Extraktionsmittel angereicherten Stromes 6, als Kopfprodukt, der den mindestens einen Katalysator enthält, und eines an Extraktionsmittel abgereicherten Stromes 7, der Katalysatorabbauprodukte, den mindestens einen Promotor, Pentennitrile, Adipodinitril und Methylglutarnitril enthält,

Der Verfahrensschritt (c) kann in jeder geeigneten, dem Fachmann bekannten Apparatur durchgeführt werden. Die Extraktion des Verfahrensschritts (c) findet vorzugsweise in Gegenstrom-Extraktionskolonnen, Mixer-Settler-Kaskaden oder Kombinationen von Mixer-Settler-Kaskaden mit Kolonnen statt. Besonders bevorzugt ist die Verwendung von Gegenstrom-Extraktionskolonnen, die insbesondere mit Blechpackungen als dispergierende Elemente ausgestattet sind. Dieses ist überraschend, da der Hydrocyanierungsaustrag feststoffbeladen ist. Erfindungsgemäß wurde festgestellt, dass der Nickel(II)cyanid-haltige Feststoff, der im Hydrocyanierungsschritt (a) entsteht, entgegen den Erwartungen unter den Bedingungen in der Extraktion nicht zum Aufwachsen neigt und keine merklichen Ablagerungen auf Kolonneneinbauten bildet.

In einer weiteren besonders bevorzugten Ausführungsform wird die Gegenstromextraktion in einer kompartimentierten, gerührten Extraktionskolonne ausgeführt.

In einer bevorzugten Ausführungsform wird das Extraktionsmittel als disperse Phase eingesetzt, der in Verfahrensschritt (b) erhaltene Sumpfaustrag oder der Austrag aus der Hydrocyanierung in Verfahrensschritt (a) als kontinuierliche Phase eingesetzt.

In der Extraktion kann ein Phasenverhältnis von 0,1 bis 10, berechnet als Verhältnis von Volumen des zugeführten Extraktionsmittels zu Volumen der zu extrahierenden Mischung, eingesetzt werden. In einer bevorzugten Ausführungsform wird die Extraktion mit einem Phasenverhältnis von 0,4 bis 2,5, in einer besonders bevorzugten Ausführungsform bei 0,75 bis 1,5 betrieben.

Der absolute Druck in Verfahrenschritt (c) beträgt vorzugsweise 0,1 bis 10 bar, besonders bevorzugt 0,5 bis 5 bar, insbesondere 1,0 bis 2,5 bar. Die Extraktion wird vorzugsweise bei Temperaturen von -15 bis 120 °C, besonders bevorzugt 0 bis 60 °C, insbesondere 25 bis 45 °C, durchgeführt.

In der vorliegenden Erfindung wird das Extraktionsmittel ausgewählt aus der Gruppe bestehend aus Cyclohexan, Methylcyclohexan, n-Hexan, n-Heptan, isomeren C6-, C7-, C8, C9-Cycloaliphaten, isomeren C6-, C7-, C8, C9-Isoallphaten, cis-, trans-Decahydronaphthalin und Gemischen davon.

In einer bevorzugten Ausführungsform wird als Extraktionsmittel Cyclohexan oder Methylcyclohexan oder n-Heptan eingesetzt.

Das verwendete Extraktionsmittel ist vorzugsweise wasserfrei, wobei unter wasserfrei im Sinne der vorliegenden Erfindung verstanden wird, dass das Extraktionsmittel weniger als 100 ppm, vorzugsweise weniger als 50 ppm, insbesondere weniger als 10 ppm, Wasser enthält. Das Extraktionsmittel kann durch geeignete, dem Fachmann bekannte Verfahren getrocknet werden, beispielsweise durch Adsorption oder Azeotropdestillation.

Das Extraktionsmittel wird dabei vorzugsweise in einem separaten Verfahrensschritt (j) durch Azeotropdestillation getrocknet. Dieses erfolgt vorzugsweise destillativ als Heteroazeotropdestillation. Der absolute Druck in diesem Verfahrensschritt (j) beträgt vorzugsweise 0,01 bis 10,0 bar, besonders bevorzugt 0,05 bis 5,0 bar, insbesondere 0,1 bis 1,0 bar. Die Destillation wird so durchgeführt, dass die Temperatur im Sumpf der Destillationsapparatur vorzugsweise 40 bis 250 °C, besonders bevorzugt 50 bis 180 °C, insbesondere 60 bis 150 °C, beträgt. Die Destillation wird so durchgeführt, dass die Temperatur am Kopf der Destillationsapparatur vorzugsweise 0 bis 200 °C, besonders bevorzugt 5 bis 100 °C, insbesondere 20 bis 50 °C, beträgt. In einer besonders bevorzugten Ausführungsform des erfindungsgemäßen Verfahrens werden die zuvor genannten Temperaturbereiche sowohl am Kopf als auch im Sumpf der Destillationsapparatur eingehalten.

Die Azeotropdestillation des Extraktionsmittels erfolgt vorzugsweise in einer Destillationskolonne mit insbesondere Glockenböden, strukturierten Blechpackungen, strukturierten Gewebepackungen, Dual-Flow-Böden oder Schüttungen aus Füllkörpern als trennwirksame Einbauten, gegebenenfalls in einer Trennwandkolonne mit gegebenenfalls vorhandenen Seitenabzügen, einem Phasenabscheider am flüssigen Abzug des Kopfkondensators zur Auskreisung von Wasser, mit Apparaten für getrennte Rückführung organischer Phasen als Rücklauf auf Kolonnen, sowie weitere zur Azeotropdestillation geeigneten Apparaturen.

In *Verfahrensschritt (d)* findet eine Destillation des Stromes 6 unter Erhalt eines den mindestens einen Katalysator enthaltenden Stromes 8 und eines das Extraktionsmittel enthaltenden Stromes 9 statt.

Dieser Verfahrensschritt dient im Wesentlichen zum Zurückgewinnen des Katalysators und des Extraktionsmittels.

Der Verfahrensschritt (d) kann in jeder geeigneten, dem Fachmann bekannten Apparatur durchgeführt werden. Die Destillation des Verfahrensschrittes (d) findet vorzugsweise in einer oder mehreren Verdampfungsstufen sowie Destillationskolonnen statt.

Als Einbauten für die Destillationskolonnen werden vorzugsweise strukturierte Blechpackung, strukturierte Gewebepackung, Glockenböden, Dual-flow-Böden oder Schüttungen aus Füllkörpern oder Kombinationen von zwei oder mehreren dieser Klassen von trennwirksamen Einbauten verwendet. Die Destillationskolonne des Verfahrensschrittes (d) kann mit einem oder mehreren flüssigen oder gasförmigen Seitenabzüge ausgeführt sein. Die Destillationskolonne aus Verfahrensschritt (d) kann als Trennwandkolonne mit einem oder mehreren vorhandenen gasförmigen oder flüssigen Seitenabzügen ausgeführt werden.

Die eine oder mehrere Verdampferstufen beziehungsweise die Destillationskolonne des Verfahrensschrittes (d) können insbesondere mit Fallfilmverdampfer, Dünnschichtverdampfer, Naturumlaufverdampfer, Zwangsumlaufentspannungsverdampfer und Mehrphasenwendelrohrverdampfer ausgerüstet sein.

In einer weiteren Ausführungsform des erfindungsgemäßen Verfahrens wird mindestens eine der Verdampfereinheiten von Verfahrensschritt (d) mit einem geteilten Sumpf betrieben, wobei man aus einem ersten Sumpf der betreffenden Verdampferstufe den im Verhältnis zum Sumpfabzugsstrom im Allgemeinen großen Umlaufstrom zum Verdampfer fährt, den flüssigen Ablaufstrom aus dem Verdampfer jedoch nicht direkt in den Sumpf zurückgibt, sondern in einen zweiten Sumpf, der vom ersten Sumpf getrennt ist, auffängt, aus dem zweiten Sumpf den Sumpfabzugsstrom erhält und den verbleibenden Überschuss vom Verdampferumlaufstrom in den ersten Sumpf überlaufen lässt, wobei als Sumpfabzugsstrom aus dem zweiten Sumpf eine Mischung erhalten wird, die gegenüber dem Abzug aus dem ersten Sumpf an Leichtsiedern abgereichert ist.

Der absolute Druck in Verfahrenschritt (d) beträgt vorzugsweise 0,001 bis 2,0 bar, besonders bevorzugt 0,01 bis 0,5 bar, insbesondere 0,09 bis 0,12 bar. Die Destillation wird so durchgeführt, dass die Temperatur im Sumpf der Destillationsapparatur vorzugsweise 40 bis 150°C, besonders bevorzugt 70 bis 120 °C, insbesondere 80 bis 100 °C, beträgt. Die Destillation wird so durchgeführt, dass die Temperatur am Kopf der Destillationsapparatur vorzugsweise -15 bis 100°C, besonders bevorzugt 0 bis 60 °C, insbesondere 20 bis 50 °C, beträgt. In einer besonders bevorzugten Ausführungsform des erfindungsgemäßen Verfahrens werden die zuvor genannten Temperaturbereiche sowohl am Kopf als auch im Sumpf eingehalten.

Bei der Abtrennung des Extraktionsmittels zur Rückgewinnung des Katalysators gemäß Verfahrensschritt d) wird 3-Pentennitril als Zwischensieder zur Destillation gegeben.. Dieser Lösemittelwechsel hat zum einen den Vorteil, dass eine effektive Abreicherung des Extraktionsmittels aus dem hochsiedenden Katalysatorstrom bei Verdampfertemperaturen möglich wird, die niedrig genug sind, um den jeweilig eingesetzten Nickelkatalysator und insbesondere den Chelatliganden nicht thermisch zu schädigen, wobei der Druck noch hoch genug ist, um das im Vergleich zu den Katalysatorbestandteilen vergleichsweise leichtsiedende Extraktionsmittel am Kopf der Verdampferstufe oder Destillationskolonne noch bei üblichen Kühlwassertemperaturen von 25 bis 50 °C kondensieren zu können. Der Lösungsmittelwechsel hat zudem den Vorteil, dass die Fließfähigkeit und die Einphasigkeit der Katalysatorlösung sichergestellt wird, da, abhängig von Temperatur und Restgehalt an Extraktionsmittel - ohne den Zusatz von 3-Pentennitril - gegebenenfalls Katalysatorbestandteile auskristallisieren können. Dabei wirkt sich 3-Pentennitril, das beispielsweise von den Extraktionsmitteln Cyclohexan oder Methylcyclohexan je nach Druckverhältnissen nur schwer oder wegen Minimumsdampfdruckazeotropbildung gar nicht vollständig abtrennbar ist, bei einem Anteil von vorzugsweise bis 10 Gew.-%, besonders bevorzugt bis 5 Gew.-%, insbesondere bis 1 Gew.-%, bezogen auf die Gesamtmenge des Extraktionsmittelzulaufstroms zur Extraktionskolonne in Verfahrensschritt (c), nicht störend auf das erfindungsgemäße Verfahren aus.

Der in Verfahrensschritt (d) erhaltene Strom 9, enthaltend das Extraktionsmittel, wird in einer bevorzugten Ausführungsform des erfindungsgemäßen Verfahrens zumindest teilweise in den Extraktionsschritt (c) zurückgeführt. Dabei wird der recyclierte Strom 9 vorzugsweise vor dem Extraktionsschritt (c) getrocknet, beispielsweise in dem oben beschriebenen Verfahrensschritt (j), so dass der Wassergehalt in diesem Strom vorzugsweise weniger als 100 ppm, besonders bevorzugt weniger als 50 ppm, insbesondere weniger als 10 ppm, beträgt.

Der in Verfahrensschritt (d) erhaltene Strom 8, enthaltend den Katalysator, wird in einer weiteren bevorzugten Ausführungsform des erfindungsgemäßen Verfahrens zumindest teilweise in die Hydrocyanierung des Verfahrensschritts (a) zurückgeführt. In einer bevorzugten Ausführungsform des erfindungsgemäßen Verfahrens beträgt der Anteil an Extraktionsmittel in dem Strom 8 vorzugsweise weniger als 10 Gew.-%, besonders bevorzugt weniger als 5 Gew.-%, insbesondere weniger als 1 Gew.-%, bezogen auf die Gesamtmenge von Strom 8.

Da die Abtrennung des Extraktionsmittels von dem Katalysator somit nicht zwingend quantitativ erfolgt, kann sich gegebenenfalls ergeben, dass noch in Strom 8 enthaltenes Extraktionsmittel ebenfalls in die Hydrocyanierungstufe zurückgeführt wird. Dieses Extraktionsmittel wird dann in der sich anschließenden oben beschriebenen Verfahrensstufe (b) im Wesentlichen in den Strom 4, enthaltend Pentennitrile, überführt und sich dort aufpegeln. Falls Extraktionsmittel in den Strom 4 gelangen sollte, ist es somit vorteilhaft, diesen Strom 4 in einem weiteren Verfahrensschritt (k) durch Destillation von dem Extraktionsmittel zu befreien. Der Verfahrensschritt (k) kann in jeder geeigneten, dem Fachmann bekannten Apparatur durchgeführt werden. Die Destillation des Verfahrensschrittes (k) findet vorzugsweise in einer oder mehreren Destillationskolonnen statt. Als Einbauten für die Destillationskolonnen werden vorzugsweise strukturierte Blechpackungen, strukturierte Gewebepackungen, Glockenböden, Dual-flow-Böden oder Schüttungen aus Füllkörpern oder Kombinationen von zwei oder mehreren dieser Klassen von trennwirksamen Einbauten verwendet. Die Destillationskolonne des Verfahrensschrittes (k) kann mit einem oder mehreren flüssigen oder gasförmigen Seitenabzüge ausgeführt sein. Die Destillationskolonne aus Verfahrensschritt (d) kann als Trennwandkolonne mit einem oder mehreren vorhandenen gasförmigen oder flüssigen Seitenabzügen ausgeführt werden.

Hierdurch bilden sich die Ströme 21, enthaltend Pentennitrile, und 22, enthaltend das Extraktionsmittel. Der Strom 21 wird vorzugsweise in den oben beschriebenen gegebenenfalls durchzuführenden Verfahrensschritt (i) zurückgeführt, um den Strom von gegebenenfalls vorhandenem cis-2-Pentennitril und (E)-2-Methyl-2-butennitril zu befreien. Der Strom 22, enthaltend das Extraktionsmittel, wird vorzugsweise in die Extraktion des Verfahrensschrittes (c) zurückgeführt.

Der so erhaltene Strom 22, enthaltend das Extraktionsmittel, wird in einer bevorzugten Ausführungsform des erfindungsgemäf3en Verfahrens zumindest teilweise in den Extraktionsschritt (c) zurückgeführt. Dabei wird der recyclierte Strom 22 vorzugsweise vor dem Extraktionsschritt (c) getrocknet, beispielsweise in dem oben beschriebenen Verfahrensschritt (j), so dass der Wassergehalt in diesem Strom vorzugsweise weniger als 100 ppm, besonders bevorzugt weniger als 50 ppm, insbesondere weniger als 10 ppm, beträgt.

In einer weiteren Ausführungsform werden die Ströme 9 und 22 vor ihrer Rückführung in die Extraktion des Verfahrensschrittes (c), gegebenenfalls in einer Apparatur, getrocknet.

Der absolute Druck in dem optionalen Verfahrensschritt (k) beträgt vorzugsweise 0,01 bis 10,0 bar, besonders bevorzugt 0,05 bis 5,0 bar, insbesondere 0,1 bis 1,0 bar. Die Destillation wird so durchgeführt, dass die Temperatur im Sumpf der Destillationsapparatur vorzugsweise 40 bis 250 °C, besonders bevorzugt 50 bis 180 °C, insbesondere 60 bis 150 °C, beträgt. Die Destillation wird so durchgeführt, dass die Temperatur am Kopf der Destillationsapparatur vorzugsweise 0 bis 200 °C, besonders bevorzugt 15 bis 180 °C, insbesondere 20 bis 150 °C, beträgt. In einer besonders bevorzugten Ausführungsform des erfindungsgemäßen Verfahrens werden die zuvor genannten Temperaturbereiche sowohl am Kopf als auch im Sumpf der Destillationsapparatur eingehalten.

In *Verfahrensschritt (e)* findet eine Destillation des Stromes 7 unter Erhalt eines Stromes 10 als Sumpfprodukt, der Katalysatorabbauprodukte, den mindestens einen Promotor, Pentennitrile, Adipodinitril und Methylglutarnitril enthält, und eines das Extraktionsmittel enthaltenden Stromes 11 als Kopfprodukt statt.

Der Verfahrensschritt (e) kann in jeder geeigneten, dem Fachmann bekannten Apparatur durchgeführt werden. Die Destillation des Verfahrensschrittes (e) findet vorzugsweise in einer oder mehreren Verdampferstufen beziehungsweise ein oder mehreren Destillationskolonnen statt. Als Einbauten für die Destillationskolonnen werden vorzugsweise strukturierte Blechpackungen, strukturierte Gewebepackungen, Glockenböden, Dual-flow-Böden oder Schüttungen aus Füllkörpern oder Kombinationen von zwei oder mehreren dieser Klassen von trennwirksamen Einbauten verwendet. Die Destillationskolonne des Verfahrensschrittes (e) kann mit einem oder mehreren flüssigen oder gasförmigen Seitenabzügen ausgeführt sein. Die Destillationskolonne aus Verfahrensschritt (e) kann als Trennwandkolonne mit einem oder mehreren vorhandenen gasförmigen oder flüssigen Seitenabzügen ausgeführt werden.

Die Verdampfereinheiten für die eine oder mehreren Verdampferstufen beziehungsweise ein oder mehreren Destillationskolonnen können insbesondere mit Fallfilmverdampfer, Dünnschichtverdampfer, Naturumlaufverdampfer, Zwangsumlaufentspannungsverdampfer und Mehrphasenwendelrohrverdampfer ausgerüstet sein.

In einer bevorzugten Ausführungsform des erfindungsgemäßen Verfahrens wird mindestens eine der einen oder mehreren Verdampfereinheiten von Verfahrensschritt (e) mit einem geteilten Sumpf betrieben, wobei man aus einem ersten Sumpf der betreffenden Verdampferstufe den im Verhältnis zum Sumpfabzugsstrom im Allgemeinen großen Umlaufstrom zum Verdampfer fährt, den flüssigen Ablaufstrom aus dem Verdampfer jedoch nicht direkt in den Sumpf zurückgibt, sondern in einen zweiten Sumpf, der vom ersten Sumpf getrennt ist, auffängt, aus dem zweiten Sumpf den Sumpfabzugsstrom erhält und den verbleibenden Überschuss vom Verdampferumlaufstrom in den ersten Sumpf überlaufen lässt, wobei als Sumpfabzugsstrom aus dem zweiten Sumpf eine Mischung erhalten wird, die gegenüber dem Abzug aus dem ersten Sumpf an Leichtsiedern abgereichert ist.

Der absolute Druck in Verfahrenschritt (e) beträgt vorzugsweise 0,1 bis 10,0 bar, besonders bevorzugt 0,5 bis 5,0 bar, insbesondere 0,15 bis 0,2 bar. Die Destillation wird dabei so durchgeführt, dass die Temperatur im Sumpf der Destillationsapparatur vorzugsweise 40 bis 250 °C, besonders bevorzugt 50 bis 200 °C, insbesondere 100 bis 180 °C, beträgt. Ferner wird die Destillation so durchgeführt, dass die Temperatur am Kopf der Destillationsapparatur vorzugsweise -15 bis 150 °C, besonders bevorzugt 0 bis 60 °C, insbesondere 20 bis 50 °C, beträgt. In einer besonders bevorzugten Ausführungsform des erfindungsgemäßen Verfahrens werden die zuvor genannten Temperaturbereiche sowohl am Kopf als auch im Sumpf eingehalten.

Der Strom 11, enthaltend das Extraktionsmittel, wird vorzugsweise in die Extraktion des Verfahrensschritts (c) zurückgeführt. Dabei wird der Strom 11, vor seiner Rückführung in die Extraktion des Verfahrensschrittes (c) vorzugsweise getrocknet, so dass der Wassergehalt in diesen Strömen vorzugsweise weniger als 100 ppm, besonders bevorzugt weniger als 50 ppm, insbesondere weniger als 10 ppm, beträgt. Dieses kann beispielsweise in dem Verfahrensschritt (j) erfolgen.

In einer bevorzugten Ausführungsform A des erfindungsgemäßen Verfahrens wird die Destillation im Verfahrensschritt (e) so durchgeführt, dass der erhaltene Strom 10 weniger als 10 Gew.-%, besonders bevorzugt weniger als 1 Gew.-%, insbesondere weniger als 0,1 Gew.-%, Extraktionsmittel enthält.

In einer weiteren bevorzugten Ausführungsform B des erfindungsgemäßen Verfahrens umfasst der Strom 11 weniger als 10 Gew.-%, besonders bevorzugt weniger als 5 Gew.-%, insbesondere weniger als 2 Gew.-%, Pentennitrile.

In einer besonders bevorzugten Ausführungsform C werden die Spezifikationen gemäß Ausführungsform A und B erreicht.

In einer weiteren bevorzugten Ausführungsform D des erfindungsgemäßen Verfahrens wird die Destillation im Verfahrensschritt (e) so durchgeführt, dass der erhaltene Strom 10 weniger als 10 Gew.-%, besonders bevorzugt weniger als 5 Gew.-%, insbesondere weniger als 1 Gew.-%, Pentennitrile enthält. Falls diese Spezifikation des Stromes 10 erreicht wird, ist es möglich, den Strom 10 unter Ausschluss des Verfahrensschrittes (f) direkt in den Verfahrensschritt (g) zu führen. Diese Spezifikation wird vorzugsweise dadurch erreicht, dass in Verfahrensschritt (e) eine dem temperaturabhängigen Dampfdruck von Pentennitril angepasste Kombination aus Verdampfungstemperatur und Destillationsdruck verwendet wird, so dass über Sumpf Pentennitrile deutlich abgereichert werden und über Kopf ein Gemisch von Pentennitril und dem jeweilig verwendeten Extraktionsmittel erhalten wird. In einer bevorzugten Ausführungsform wird dieses Gemisch von Substanzen mit merklich unterschiedlichen Kondensationspunkten in einem Verfahren für geschlossene Kondensation kondensiert. Durch die geschlossene Kondensation kann der Strom 11 im Vergleich zur üblichen offenen Kondensation bei deutlich höheren Temperaturen vollständig kondensiert werden, beziehungsweise ein im Vergleich zur offenen Kondensation bei gleicher Kühlmediumstemperatur deutlich niedrigerer Kolonnendruck und damit bei gleicher Sumpftemperatur ein geringerer Gehalt an Leichtsiedern im Sumpfabzugsstrom eingestellt werden. Diese Ausführungsform führt dazu, dass eine Destillationsstufe zur Rückführung von Pentennitrilen aus Strom 7 eingespart werden kann.

Die Höhe der üblicherweise vorgefundenen Gehalte von Pentennitrilen in Strom 7 nach der Extraktion machen eine Abtrennung und Rückführung für die wirtschaftlcihe Ausübung des Verfahrens notwendig. Die geschlossene Kondensation kann in allen weiteren, dem Fachmann bekannten und geeigneten Weisen ausgeführt werden. Eine geeignete Ausführungsform ist die Durchführung der Kondensation in einem stehenden Rohrbündelkondensator, wobei das Produkt in den Rohren geführt wird und der brüdenseitige Eintritt in die Rohre mit einem im Vergleich zur Kopfabzugsmenge großen Umwälzstrom aus dem zum Kondensator gehörigen Kondensatsammelbehälter gespült wird. Eine weitere geeingete Ausführungsform ist die Durchführung der Kondensation in einem Rohrbündelwärmetauscher, wobei das Produkt im Mantelraum geführt wird und der Mantelraum mit einem Umwälzstrom aus dem zum Kondensator zugehörigen Kondensatsammelbehälter gespült wird. Eine besonders bevorzugte Ausführungsform der geschlossenen Kondensation ist die Verwendung eines Direktkondensators, der als ein auf die Kolonne in Verfahrensschritt (e) aufgesetzter Kolonnenschuss mit Totalfangtasse, einem oder mehreren flüssigen Abzügen für einen Umpumpkreis, Wärmetauscher im Umpumpkreis zum Abführen der Kondensationswärme und Rückführung des gekühlten Umlaufstromes in den Kolonnenschuss ausgerüstet ist. In einer bevorzugten Ausführungsform E des erfindungsgemäßen Verfahrens umfasst der Strom 11 1 Gew.-% bis 90 Gew.-%, besonders bevorzugt 5 bis 80 Gew.-%, insbesondere 10 bis 60 Gew.-%, Pentennitrile.

In einer weiteren besonders bevorzugten Ausführungsform F werden die Spezifikationen gemäß Ausführungsform D und E erreicht.

In *Verfahrensschritt (f)* findet eine Destillation des Stromes 10 unter Erhalt eines Stromes 12 als Sumpfprodukt, der Katalysatorabbauprodukte, den mindestens einen Promotor, Adipodinitril und Methylglutarnitril enthält, und eines Pentennitrile enthaltenden Stromes 13 als Kopfprodukt statt.

Der Verfahrensschritt (f) kann in jeder geeigneten, dem Fachmann bekannten Apparatur durchgeführt werden. Die Destillation des Verfahrensschrittes (f) findet vorzugsweise in einer oder mehreren Verdampferstufen beziehungsweise ein oder mehreren Destillationskolonnen statt. Als Einbauten für die Destillationskolonnen werden vorzugsweise strukturierte Blechpackungen, strukturierte Gewebepackungen, Glockenböden, Dual-flow-Böden oder Schüttungen aus Füllkörpern oder Kombinationen von zwei oder mehreren dieser Klassen von trennwirksamen Einbauten verwendet. Die Destillationskolonne des Verfahrensschrittes (f) kann mit einem oder mehreren flüssigen oder gasförmigen Seitenabzügen ausgeführt sein. Die Destillationskolonne aus Verfahrensschritt (f) kann als Trennwandkolonne mit einem oder mehreren vorhandenen gasförmigen oder flüssigen Seitenabzügen ausgeführt werden.

Die Verdampfereinheiten für die eine oder mehrere Verdampferstufen beziehungsweise ein oder mehrere Destillationskolonnen können insbesondere mit Fallfilmverdampfer, Dünnschichtverdampfer, Naturumlaufverdampfer, Zwangsumlaufentspannungsverdampfer und Mehrphasenwendelrohrverdampfer ausgerüstet sein.

In einer bevorzugten Ausführungsform des erfindungsgemäßen Verfahrens wird mindestens eine der einen oder mehreren Verdampfereinheiten von Verfahrensschritt (f) mit einem geteilten Sumpf betrieben, wobei man aus einem ersten Sumpf der betreffenden Verdampferstufe den im Verhältnis zum Sumpfabzugsstrom im Allgemeinen großen Umlaufstrom zum Verdampfer fährt, den flüssigen Ablaufstrom aus dem Verdampfer jedoch nicht direkt in den Sumpf zurückgibt, sondern in einen zweiten Sumpf, der vom ersten Sumpf getrennt ist, auffängt, aus dem zweiten Sumpf den Sumpfabzugsstrom erhält und den verbleibenden Überschuss vom Verdampferumlaufstrom in den ersten Sumpf überlaufen lässt, wobei als Sumpfabzugsstrom aus dem zweiten Sumpf eine Mischung erhalten wird, die gegenüber dem Abzug aus dem ersten Sumpf an Leichtsiedern abgereichert ist.

Der absolute Druck in Verfahrenschritt (f) beträgt vorzugsweise 0,001 bis 1,0 bar, besonders bevorzugt 0,005 bis 0,1 bar, insbesondere 0,01 bis 0,05 bar. Die Destillation wird so durchgeführt, dass die Temperatur im Sumpf der Destillationsapparatur vorzugsweise 40 bis 250 °C, besonders bevorzugt 50 bis 200 °C, insbesondere 100 bis 180 °C, beträgt. Die Destillation wird so durchgeführt, dass die Temperatur am Kopf der Destillationsapparatur vorzugsweise -15 bis 150 °C, besonders bevorzugt 0 bis 60 °C, insbesondere 20 bis 50 °C, beträgt. In einer besonders bevorzugten Ausführungsform des erfindungsgemäßen Verfahrens werden die zuvor genannten Temperaturbereiche sowohl am Kopf als auch im Sumpf eingehalten.

Der Gehalt an Pentennitrilen in Strom 12 beträgt vorzugsweise weniger als 10 Gew.-%, besonders bevorzugt weniger als 2 Gew.%, insbesondere weniger als 0,5 Gew.-%.

Der in Verfahrensschritt (f) erhaltene Strom 13 enthält im Allgemeinen trans-3-Pentennitril, cis-3-Pentnnitril, 4-Pentennitril, trans-2-Pentnnitril, cis-2-Pentennitril und (E)-2-Methyl-2-butennitril. In einer besonders bevorzugten Ausführungsform wird dieser Strom 13 zumindest teilweise unter Erhalt eines an trans-3-Pentennitril, cis-3-Pentnnitril, 4-Pentennitril, trans-2-Pentnnitril, cis-2-Pentennitril und (E)-2-Methyl-2-butennitril abgereicherten Stromes 22 und eines an trans-3-Pentennitril, cis-3-Pentennitril, 4-Pentennitril, trans-2-Pentennitril, cis-2-Pentennitril und (E)-2-Methyl-2-butennitril angereicherten Stromes 23 in einem weiteren Verfahrensschritt (I) destilliert. Der an cis-2-Pentennitril und (E)-2-Methyl-2-butennitril abgereicherten Strom 22 wird vorzugsweise in die Hydrocyanierung des Verfahrensschrittes (a) zurückgeführt.

In einer besonders bevorzugten Ausführungsform des erfindungsgemäßen Verfahrens ist vorgesehen, dass sowohl der Strom 4 als auch der Strom 13, gegebenenfalls in der gleichen Apparatur aus Verfahrensschritt (i), zur Abreicherung an cis-2-Pentennitril und (E)-2-Methyl-2-butennitril destilliert wird. Der sich hieraus ergebende an cis-2-Pentennitril und (E)-2-Methyl-2-butennitril abgereicherte Strom 19 wird gegebenenfalls zumindest teilweise in die Hydrocyanierung zurückgeführt

In *Verfahrensschritt (g)* findet eine Destillation des Stromes 12 unter Erhalt eines Stromes 14 als Sumpfprodukt, enthaltend Katalysatorabbauprodukte und den mindestens einen Promotor, und eines Stromes 15 als Kopfprodukt, enthaltend Adipodinitril und Methylglutarnitril, statt.

Der Verfahrensschritt (g) kann in jeder geeigneten, dem Fachmann bekannten Apparatur durchgeführt werden. Die Destillation des Verfahrensschrittes (g) findet besonders bevorzugt in einer oder mehreren Destillationskolonnen statt. Die Kolonnen können mit einem oder mehreren Seitenabzügen ausgestattet sein. Als Einbauten für die Destillationskolonnen werden vorzugsweise strukturierte Blechpackungen, strukturierte Gewebepackungen, Glockenbödenen, Dual-flow-Böden oder Schüttungen aus Füllkörpern oder Kombinationen von zwei oder mehreren dieser Klassen von trennwirksamen Einbauten verwendet.

Die Destillation des Verfahrensschritts (g) findet besonders bevorzugt als Verdampfung mit einer oder mehreren hintereinandergeschalteten Stufen und Kondensation mit dem Fachmann an sich bekannten Verdampfern statt. Beispiele für geeignete Verdampfer für die eine oder mehreren Verdampferstufen sind Fallfilmverdampfer, Naturumlaufverdampfer, Zwangsumlaufentspannungsverdampfer, Dünnschichtverdampfer, Kurzwegverdampfer und Mehrphasenwendelrohrverdampfer. Als besonders bevorzugt gelten Verdampfer, die für das gewünschte Erreichen des Verdampfungsgrades eine möglichst niedrige Verdampferoberflächentemperatur und eine kurze Kontaktzeit an der Verdampferoberfläche und damit geringe thermische Schädigung des einzuengenden Materials ermöglichen. Hierfür sind insbesondere Fallfilmverdampfer, Dünnschichtverdampfer, Mehrphasenwendelrohrverdampfer und Kurzwegverdampfer geeignet.

Der absolute Druck in Verfahrenschritt (g) beträgt vorzugsweise 0,0001 bis 0,5 bar, besonders bevorzugt 0,001 bis 0,05 bar, insbesondere 0,002 bis 0,01 bar. Die Destillation wird so durchgeführt, dass die Temperatur im Sumpf der Destillationsapparatur vorzugsweise 60 bis 300 °C, besonders bevorzugt 120 bis 220 °C, insbesondere 140 bis 180 °C, beträgt. Die Destillation wird so durchgeführt, dass die Temperatur am Kopf der Destillationsapparatur vorzugsweise 5 bis 250 °C, besonders bevorzugt 40 bis 180 °C, insbesondere 60 bis 120 °C, beträgt. In einer besonders bevorzugten Ausführungsform des erfindungsgemäßen Verfahrens werden die zuvor genannten Temperaturbereiche sowohl am Kopf als auch im Sumpf der Destillationsapparatur eingehalten.

In Verfahrensstufe (g) wird der Strom 14, der Katalysatorabbauprodukte und den mindestens einen Promotor enthält, abgetrennt. Dieser Strom ist im Allgemeinen zähfließend, so dass es sich als vorteilhaft herausgestellt hat, diesen Strom durch Flüssigkeitszugabe fließfähiger zu machen. Hierfür eignet sich beispielsweise das in dem folgenden Verfahrensschritt (h) erhaltene Methylglutarnitril. In einer bevorzugten Ausführungsform der vorliegenden Erfindung wird somit der Verfahrensschritt (g) als zweistufige Destillation ausgeführt, indem der in Verfahrensschritt (g) erhaltene Strom 14 in einem nachgeschaltetem Verfahrensschritt (m) ausgequetscht wird und der ausgequetschte Strom 14 mit einem Teil des in Verfahrensschritt (h) erhaltenen Methylglutarnitril-Stroms 17 verdünnt wird. In einer weiteren bevorzugten Ausführungsform wird der in Verfahrensschritt (m) bei einem absoluten Druck von 0,0001 bis 0,5 bar durch Destillation bei einer Temperatur im Sumpf der Destillationsapparatur von 60 bis 350 °C erhaltene Strom mit Pentennitril-Isomeren verdünnt, besonders bevorzugt mit zumindest einem Teil von Strom (19).

Zudem sind als Verdünnungsmittel für Strom 14 höher als Adipodinitril siedende Stoffe geeignet, insofern sie die Fließfähigkeit erhöhen.

Der Verfahrensschritt (m) kann in jeder geeigneten, dem Fachmann bekannten Apparatur durchgeführt werden. Bevorzugt sind Dünnschichtverdampfer, Zwangsumlaufentspannungsverdampfer oder Mehrphasenwendelrohrverdampfer. Beispiele für geeignete Verdampfer für die eine oder mehreren Verdampferstufen sind Fallfilmverdampfer, Naturumlaufverdampfer, Zwangsumlaufentspannungsverdampfer, Dünnschichtverdampfer, Kurzwegverdampfer und Mehrphasenwendelrohrverdampfer. Als besonders bevorzugt gelten Verdampfer, die das für das gewünschte Erreichen des Verdampfungsgrades unter Berücksichtigung der Viskosität des einzudampfenden Materials eine möglichst niedrige Verdampferoberflächentemperatur und eine kurze Kontaktzeit an der Verdampferoberfläche und damit geringe thermische Schädigung des einzuengenden Materials und minimierte Wertproduktverluste ermöglichen. Hierfür sind insbesondere Fallfilmverdampfer, Dünnschichtverdampfer, Mehrphasenwendelrohrverdampfer und Kurzwegverdampfer geeignet.

Der absolute Druck in Verfahrensschritt (m) beträgt 0,0001 bis 0,5 bar, bevorzugt 0,001 bis 0,05 bar, insbesondere 0,002 bis 0,01 bar. Die Destillation wird so durchgeführt, dass die Temperatur im Sumpf der Destillationsapparatur 60 bis 350 °C, bevorzugt 120 bis 250 °C, insbesondere 160 bis 220 °C, beträgt. Der Druck in Verfahrensschritt (m) wird in einer besonders bevorzugten Ausführungsform innerhalb des zuvor beschriebenen Bereichs und gleichzeitig kleiner als in Verfahrensschritt (g) eingestellt.

In *Verfahrensschritt (h)* findet eine Destillation des Stromes 15 unter Erhalt eines Adipodinitril enthaltenden Stromes 16 als Sumpfprodukt und eines im Wesentlichen Methylglutarnitril enthaltenden Stromes 17 als Kopfprodukt statt.

Der Verfahrensschritt (h) kann in jeder geeigneten, dem Fachmann bekannten Apparatur durchgeführt werden. Die Destillation des Verfahrensschrittes (h) findet vorzugsweise in einer oder mehreren Destillationskolonnen statt. Als Einbauten für die Destillationskolonnen werden vorzugsweise strukturierte Blechpackungen, strukturierte Gewebepackungen, Glockenböden, Dual-flow-Böden oder Schüttungen aus Füllkörpern und deren Kombinationen als trennwirksame Einbauten verwendet. Die Destillationskolonne des Verfahrensschrittes (h) kann mit einem oder mehreren flüssigen oder gasförmigen Seitenabzüge ausgeführt sein. Die Destillationskolonne aus Verfahrensschritt (h) kann als Trennwandkolonne mit einem oder mehreren vorhandenen gasförmigen oder flüssigen Seitenabzügen ausgeführt werden.

Die Verdampfereinheiten für die eine oder mehrere Destillationskolonnen können insbesondere mit Fallfilmverdampfer, Dünnschichtverdampfer, Naturumlaufverdampfer, Zwangsumlaufentspannungsverdampfer und Mehrphasenwendelrohrverdampfer ausgerüstet sein.

Der absolute Druck in Verfahrenschritt (h) beträgt vorzugsweise 0,0001 bis 0,5 bar, besonders bevorzugt 0,005 bis 0,06 bar, insbesondere 0,01 bis 0,03 bar. Die Destillation wird dabei so durchgeführt, dass die Temperatur im Sumpf der Destillationsapparatur vorzugsweise 60 bis 300 °C, besonders bevorzugt 120 bis 220 °C, insbesondere 140 bis 180 °C, beträgt. Die Destillation wird so durchgeführt, dass die Temperatur am Kopf der Destillationsapparatur vorzugsweise 40 bis 250 °C, besonders bevorzugt 60 bis 180 °C, insbesondere 100 bis 140 °C. beträgt. In einer besonders bevorzugten Ausführungsform des erfindungsgemäßen Verfahrens werden die zuvor genannten Temperaturbereiche sowohl am Kopf als auch im Sumpf der Destillationsapparatur eingehalten.

Falls in dem Strom 15 noch Pentennitrile enthalten sein sollten, so können diese vorzugsweise über den Kopf der Destillationskolonne gewonnen werden. In diesem Fall wird der Methylglutarnitril-Strom 17 über einen Seitenabzug der Kolonne und Adipodinitril über den Sumpf der Kolonne gewonnen.

In einer weiteren Ausführungsform des erfindungsgemäßen Verfahrens wird der Strom 16, enthaltend Adipodinitril, an einem Seitenabzug gewonnen. Bevorzugt wird die Entnahme in Form eines gasförmigen Seitenabzugs unterhalb der Zulaufstelle vorgenommen. Über Sumpf wird dann eine Mischung 23 erhalten, enthaltend im Wesentlichen Adipodinitril und hochsiedende phosphorhaltige Komponenten, die trotz niedrigen Dampfdrucks über die Verfahrensstufe (g) und gegebenenfalls (m) in den Strom 15 gelangen.

In einer weiteren Ausführungsform des erfindungsgemäßen Verfahrens wird der Strom 17, enthaltend Methylglutarnitril, als Zwischensieder in Verfahrensschritt (e) zurückgeführt. In einer weiteren Ausführungsform des erfindungsgemäßen Verfahrens wird der Strom 17, enthaltend Methylglutarnitril, als Zwischensieder in Verfahrensschritt (f) zurückgeführt. In einer weiteren besonderen Ausführungsform wird der Strom 17, enthaltend Methylglutarnitril, als Zwischensieder ganz oder teilweise gleichzeitig in die Verfahrensschritte (e) und/oder (f) zurückgeführt. Durch das Zurückführen des Stromes 17 in die Verfahrenschritte (e) und /oder (f) wird im Wesentlichen erreicht, dass die Sumpftemperturen in den Verfahrensschritten (e) und/oder (f) niedrig sind, so dass die gegebenenfalls noch vorhandenen 3-Pentennitrile im Wesentlichen nicht mit dem Promotor, beispielsweise Zinkchlorid, reagieren.

In einer weiteren Ausführungsform werden andere geeignete Mittelsieder vom Kopf oder von einem Seitenabzug der Kolonne von Verfahrensschritt (h) in die Verfahrensschritte (e) und/oder (f) zurückgeführt. Geeignet sind Stoffe, die keine Dampfdurckminimumsazeotrope mit Pentennitrilen oder dem Extraktionsmittel und seinen Komponenten bilden und bei den jeweiligen Sumpftempereaturen von Verfahrensschritten (e) und/oder (f) einen Dampfruck haben, der zwischen dem der Pentennitrile und dem von Methylglutarnitril liegt, beispielsweise Alkylphenole wie Kresolisomere oder tert.-Butylphenol, die als Abbauprodukte der Katalysatorkomponenten in Spuren im Verfahren gebildet werden und im Kopfbereich der Kolonnen von Verfahrensschrit (h) angereichert werden.

In einer weiteren Ausführungsform des erfindungsgemäßen Verfahrens wird der Strom 17, enthaltend im Wesentlichen Methylglutarnitril, zur Verdünnung des Stromes 14 verwendet.

Die durch das zuvor beschriebene Verfahren erhältliche Adipodinitril weist eine Reinheit auf, die durch die Nebenkomponenten von vorzugsweise 1 Gew.-ppm bis 1 Gew.-%, besonders bevorzugt 5 bis 1000 Gew.-ppm, insbesondere 10 bis 500 Gew.-ppm, Methylglutarnitril, sowie vorzugsweise 0,01 bis 1000 Gew.-ppm Methylglutarnitril, besonders bevorzugt 0,1 bis 500 Gew.-ppm, insbesondere 1 bis 20 Gew.-ppm, Phosphorverbindungen berechnet als elementarer Phosphor, nachweisbar als Summe von flüchtigen und nicht flüchtigen Phosphorverbindungen, charakterisiert ist.

Die durch das zuvor beschriebene Verfahren erhältliche Methylglutarnitril weist eine Reinheit von vorzugsweise 50 bis 100 Gew.-%, besonders bevorzugt 80 bis 99 Gew.-%, insbesondere 90 bis 98 Gew.%, auf, berechnet als Summe von Methylglutarnitril und dem Isomeren Ethylbernsteinsäuredinitril, wobei letzteres in der Hydrocyanierung parallel zu Methylglutarnitril und von Methylglutarnitril nahezu untrennbar erhalten wird.

Im Sinne der vorliegenden Anmeldung ist unter dem Begriff Methylglutarnitril stets Methylglutarnitril oder ein Gemisch aus Methylglutarnitril und Ethylbernsteinsäuredinitril zu verstehen.

Da der Promotor bereits in Verfahrensschritt (g) von Adipodinitril und Methylglutarnitril abgetrennt wurde, ist es nicht zwingend, dass die Adipodinitril-/Methylglutarnitril-Destillation in Apparaturen aus chloridbeständigen Materialien erfolgt. Die Ausführung der Apparaturen für die Verfahrensschritte (e), (f), (g) und (m) erfordert beim Einsatz von chloridhaltigen Lewissäuren als Promotor den Einsatz von hochwertigen Werkstoffen, Wandbeschichtungen oder sonstige Einrichtungen zum Schutz gegen Korrosion, die bei den angewendeten Temperaturen beständig gegen Angriff durch chloridhaltige Medien sind. Gleiches gilt für die Verwendung bromidhaltiger Promotoren.

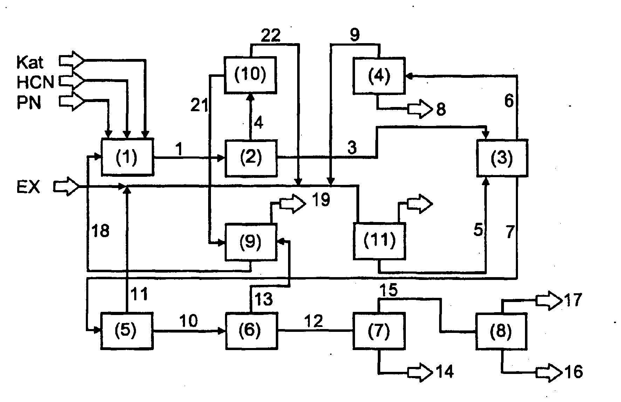

### Figur 1 zeigt:

In dem Reaktor (1) werden ein Nickel(0)-Katalysator (Kat), Pentennitrile (PN), die aus einer Hydrocyanierung von Butadien stammen, und Cyanwasserstoff (HCN) eingespeist. Weitere Bestandteile sind 3-Pentennitrile und gegebenenfalls Extraktionsmittel (EX) in Strom 18, die in dem erfindungsgemäßen Verfahren recycliert werden. An dem Nickel(0)-Katalysator findet eine Hydrocyanierung von Pentennitrilen mit Cyanwasserstoff statt. Der hieraus resultierende Strom 1, der Pentennitrile, den mindestens einen Katalysator, Katalysatorabbauprodukte, den mindestens einen Promotor, Extraktionsmittel, Adipodinitril und Methylglutarnitril aus der Hydrocyanierung enthält, wird in eine Destillationskolonne (2) überführt.

Dort findet eine destillative Abtrennung über Kopf der Kolonne von Pentennitrilen und von vorhandenem Extraktionsmittel statt (Strom 4). Das Sumpfprodukt (Strom 3) dieser Destillationskolonne enthält im Wesentlichen den Katalysator, Katalysatorabbauprodukte, den mindestens einen Promotor, Adipodinitril und Methylglutarnitril und ist an Pentennitrilen abgereichert.

Der über Kopf der Destillationskolonne (2) gewonnene Strom 4, enthaltend Pentennitrile und das vorhandene Extraktionsmittel, wird in eine weitere Destillationskolonne überführt. Dort wird als Kopfprodukt das Extraktionsmittel gewonnen, was später zur weiteren Extraktion verwendet wird (Strom 22). Als Sumpfprodukt dieser Kolonne (10) werden Pentennitrile erhalten (Strom 21). Dieser Sumpfstrom an Pentennitrilen wird anschließend in eine weitere Kolonne (9) überführt, in der eine Trennung in 3-Pentennitril (Strom 18) und (E)-2-Methyl-2-butennitril und cis-2-Pentennitril (Strom 19) erfolgt. Das 3-Pentennitril wird in die Hydrocyanierung des Reaktors (1) zurückgeführt und kann Extraktionsmittel enthalten (Strom 18).

Das Sumpfprodukt der Destillationskolonne (2) wird anschließend in eine Extraktionskolonne überführt (Strom 3). Hier erfolgt eine Extraktion des Stromes 3 mit einem Extraktionsmittel unter Ausbildung eines Stromes 6, der über den Kopf der Extraktionskolonne (3) abgezogen wird. Dieser Strom 6 wird in eine weitere Destillationskolonne (4) überführt, in der eine Auftrennung in einen Strom 8 als Sumpfprodukt, der den Katalysator enthält, und einem Kopfstrom 9, der das Extraktionsmittel enthält, bewirkt wird. Das so erhaltene Extraktionsmittel des Stromes 9 wird anschließend in eine Extraktionsmittel-Trockenkolonne (11) überführt, in dem das trockene Extraktionsmittel als Sumpfprodukt entnommen wird.

Der aus der Destillationskolonne (3) stammende Strom 7, der in der Destillationskolonne (3) als Sumpfprodukt erhalten wird, wird anschließend in eine weitere Destillationskolonne (5) überführt.

In dieser Destillationskolonne (5) wird über Kopf noch vorhandenes Extraktionsmittel als Strom 11 abgeführt, das ebenfalls in die Extraktionsmittel-Trockenkolonne (11) überführt wird.

Als Sumpfprodukt wird aus der Kolonne (5) der Strom 10 entnommen. Dieser Strom 10 wird in eine weitere Destillationskolonne (6) überführt, in der als Kopfprodukt ein Strom 13, der Pentennitril enthält, abgetrennt wird. Dieser Strom 13 wird im Anschluss in die Destillationskolonne (9) überführt.

Als Sumpfprodukt wird aus der Destillationskolonne (6) der Strom 12, enthaltend Katalysatorabbauprodukt, Promotor, Adipodinitril und Methylglutarnitril entnommen. Dieser Strom 12 wird anschließend in eine weitere Destillationskolonne (7) überführt.

In dieser Destillationskolonne (7) wird als Sumpfprodukt der Strom 14, der Nickeiabbauprodukte und den Promotor enthält, entnommen.

Als Kopfprodukt wird aus der Destillationskolonne der Strom 15 übernommen. Der Strom 15 gelangt im Anschluss hieran in eine weitere Destillationskolonne (8), in der eine Trennung in Adipodinitril, Strom 16, und Methylglutarnitril, Strom 17, erfolgt.
Ausführungsbeispiele:

In den Beispielen werden folgende Abkürzungen verwendet:
- HCN:: Cyanwasserstoff
- T3PN:: trans-3-Pentennitril
- C3PN:: cis-3-Pentennitril
- 4PN:: 4-Pentennitril
- E2M2BN:: (E)-2-Methyl-2-butennitril
- T2PN:: trans-2-Pentennitril
- C2PN:: cis-2-Pentennitril
- ADN:: Adipodinitril
- MGN:: Methylglutarnitril
- VSN:: Valeriansäurenitril
- VCH:: 4-Vinylcyclohexen

### Referenzbeispiel 1:

In Beispiel 1 wird für die Hydrocyanierung von Butadien ein Katalysatorsystem auf Basis von Nickel(0)-Komplexen mit einem Gemisch von Liganden eingesetzt. Die Ligandmischung zur Hydrocyanierung enthält ca. 60 Mol% Tri(m/p-tolyi)phosphit und 40 Mol% des Chelatphosphonits 1: 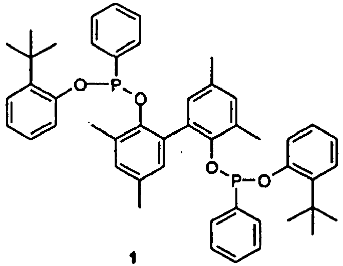 In einem Schlaufenreaktor R1 von 250 l Volumen, der mit einer Strahldüse, Impulsaustauschrohr, externem Umpumpkreislauf und Wärmetauscher zur Abfuhr der Reaktionswärme ausgestattet ist, werden folgende Ströme dosiert:
(1) 10 kg/h flüssiger, unstabilisierter, durch Destillation von Wasser befreiter Cyanwasserstoff,
(2) 39 kg/h Pentennitril, erhalten aus einem Verfahren zu Herstellung von linearem Pentennitril gemäß der DE-A-102 004 004 671, bestehend aus in Summe 97% T3PN, C3PN und 4PN sowie 2% E2M2BN,
(3) 25 kg/h Pentennitril, erhalten als Strom 18 aus der Kolonne K9 aus Schritt (9), enthaltend in Summe 81% T3PN, C3PN, 4PN sowie 3% T2PN, 1% C2PN und 3% E2M2BN.
(4) 6 kg/h Nickel(0)-Katalysatorlösung, bestehend aus 40% Ligandmischung, 2% Nickel(0) und 3% ZnCl₂, hergestellt gemäß Beispiel 13 der DE-A-103 51 002.

Das aus dem Reaktor R1 abgezogene Strom 1 enthält in Summe 40% T3PN, C3PN und 4PN sowie 47% ADN und 6% MGN, entsprechend einem Umsatz von 51% Pentennitril.

Strom 1 wird in einem Schritt 2 einer Destillationskolonne K2 zugeführt, die in Abtriebsfahrweise betrieben wird und mit Zwangsumlaufentspannungsverdampfer, einem Kopfkondensator sowie Kolonneneinbauten mit strukturierter Packung ausgestattet ist, die 15 theoretische Trennstufen erzeugen. Die Kolonne K2 wird bei 50 mbar absolutem Kopfdruck, 313 K Kopftemperatur und 353 K Sumpfabzugstemperatur betrieben.

Über Kopf der Kolonne K2 wird der Strom 4 erhalten (22 kg/h), der in Summe 81% T3PN, C3PN und 4PN sowie in Summe 8% T2PN und C2PN enthält.

Über Sumpf der Kolonne K2 werden 57 kg/h eines Stromes 3 mit einem Gehalt von in Summe 19% T3PN, C3PN und 4PN sowie in Summe 1% T2PN und C2PN erhalten. T2PN und C2PN sind im Verhältnis zu T3PN, C3PN und 4PN gegenüber dem Zulauf deutlich abgereichert. Strom 3 enthält zudem die Katalysatorkomponenten Nickel(0), die Ligandmischung und ZnCl₂ sowie Katalysatorabbauprodukte.

Strom 3 wird in einem Schritt (3) am unteren Ende einer Gegenstromextraktionskolonne K3 aufgegeben und mit 86 kg/h eines Strom 5, enthaltend 93% Methylcyclohexan sowie verschiedene Pentennitril-Isomere, extrahiert. Der am Kopf der Extraktion erhaltene Strom 6 besteht zu 91% aus dem Extraktionsmittel und enthält neben in Summe 4% T3PN, C3PN und 4PN die Katalysatorkomponenten Nickel(0) und Ligandmischung. Das Zinkchlorid verbleibt vollständig im Sumpfabzug der Extraktionskolonne in Strom 7.

Strom 6 wird in einem Schritt (4) in eine Destillationskolonne K4 gefahren, die mit Fallfilmverdampfer, einem geteilten Sumpf und Kopfkondensator sowie Kolonneneinbauten mit strukturierter Packung ausgestattet ist, die 12 theoretische Trennstufen erzeugen. Die Kolonne wird bei 100 mbar absolutem Kopfdruck, 308 K Kopftemperatur und 353 K Sumpfabzugstemperatur betrieben. Das Rücklaufverhältnis wird so eingestellt, dass in Summe 6% T3PN, C3PN und 4PN im Kopfabzugsstrom enthalten sind.

Über Kopf der Kolonne K4 wird das Extraktionsmittel zurückgewonnen, das zusammen mit einem kleinen makeup-Strom von reinem, auf Wasserkonzentration unter 10 ppm getrocknetem Methylcyclohexan als Strom 9 in die Extraktionskolonne K3 zurückgefahren wird.

Am Sumpf der Kolonne K4 wird in Strom 8 der extrahierte Katalysator als Lösung in Pentennitril gewonnen (13 kg/h). Strom 8 enthält 18% Ligandmischung, 1% Ni(0) und in Summe 68% T3PN, C3PN und 4PN. Der Methylcyclohexangehalt in Strom 8 wird in der Kolonne K4 auf 10 Gew.-ppm in Strom 8 eingestellt.

Der katalysatorreiche Strom 8 wird in der Hydrocyanierung von Butadien zu T3PN und 2M3BN eingesetzt.

Der in der Extraktionskolonne K3 erhaltene Strom 7 wird in einem Schritt (5) zu einer in Abtriebsfahrweise betriebenen Destillationskolonne K5 gefahren, die mit Zwangsumlaufentspannungsverdampfer, am Kopf mit einem Kolonnenschuss mit Totalfangtasse, Umlauf und externer Wärmeabfuhr als Direktkondensator sowie Kolonneneinbauten mit strukturierter Packung ausgestattet ist, die 5 theoretische Trennstufen erzeugen. Die Kolonne K5 wird bei 180 mbar absolutem Kopfdruck, 313 K Kopftemperatur und 453 K Sumpfabzugstemperatur betrieben.

Über Kopf der Kolonne K5 wird ein Strom 11 erhalten, der in die Destillationskolonne K4 gefahren wird. Strom 11 (22 kg/h) enthält im Wesentlichen 45% Methylcyclohexan, in Summe 48% T3PN, C3PN und 4PN sowie weitere Pentennitrile.

Über Sumpf der Kolonne K5 wird ein Strom 10 erhalten (45 kg/h), der in Summe noch 5% T3PN, C3PN und 4PN sowie 83% ADN und das Nebenprodukt MGN und zusätzlich Katalysatorabbauprodukte zusammen mit ZnCl₂ enthält.

Strom 10 wird in einem Schritt (7) in einer Verdampferstufe B7 aufgearbeitet, die mit Zwangsumlaufentspannungsverdampfer, Kopfkondensator und nachgeschaltetem Dünnschichtverdampfer B7a zum Einengen des Sumpfproduktes ausgestattet ist. Der Verdampfer wird bei einem absoluten Druck von 5 mbar und 453 K Sumpftemperatur betrieben.

Über Kopf des Verdampfers B7 wird als Roh-ADN der Strom 15 abgezogen (44 kg/h), das noch in Summe 5% T3PN, C3PN und 4PN sowie 10% MGN enthält.

Über Sumpf des Dünnschichtverdampfers B7a werden bei 473 K Sumpftemperatur eingeengte Katalysatorrückstände zusammen mit Zinkchlorid als Strom 14 erhalten, die zur Verflüssigung mit 0,5 kg/h MGN aus dem Kopf der Kolonne K8 aus Schritt (8) versetzt werden.

Strom 15 wird in einem Schritt (8) in eine Destillationskolonne K8 gefahren, die mit Dünnschichtverdampfer, einem als Teilkondensator betriebenem Kopfkondensator sowie Kolonneneinbauten mit strukturierter Packung ausgestattet ist, die 25 theoretische Trennstufen erzeugen. Die Kolonne K8 wird bei einem absoluten Druck von 25 mbar Kopfdruck, 414 K Kopftemperatur und 453 K Sumpfabzugstemperatur betrieben. Das Rücklaufverhältnis wird so eingestellt, dass im Kopfprodukt 1 % ADN enthalten sind. Die Kopfabzugsmenge der Kolonne K8 wird so geregelt, dass im Sumpfprodukt 100 Gewpppm MGN enthalten sind.

Über Sumpf der Kolonne K8 werden als Strom 16 37 kg/h ADN erhalten. Am Kopfkondensator der Kolonne K8, der bei 393 K betrieben wird, werden als flüssige Phase 3 kg/h MGN als Strom 17 erhalten, das mit 3PN, ADN und flüchtigen Katalysatorzersetzungsprodukten wie Alkylphenolen verunreinigt ist. Der gasförmige Abzug des Kondensators der Kolonne K8 wird in einem Nachkondensator bei 313 K kondensiert, wobei 4 kg/h eines Stromes 20 erhalten werden, der in Summe 54% T3PN, C3PN und 4PN sowie 41% MGN enthält.

Der Strom 4 aus der Kolonne K2 wird in Schritt (9) in einer Destillationskolonne K9 aufgearbeitet, die mit Umlaufverdampfer, Kopfkondensator sowie Kolonneneinbauten mit strukturierter Packung ausgestattet ist, die 35 theoretische Trennstufen erzeugen. Die Kolonne von Schritt (9) wird bei einem absoluten Druck von 46 mbar Kopfdruck, 313 K Kopftemperatur und 338 K Sumpfabzugstemperatur betrieben. Das Rücklaufverhältnis wird so eingestellt, dass im Kopfprodukt noch 0,5% T3PN enthalten sind.

Über Kopf der Kolonne K9 wird der Strom 19 erhalten (1,4 kg/h), der 65% C2PN und 29% E2M2BN sowie geringe Mengen VCH und VSN enthält.

Über Sumpf der Kolonne K9 wird der Strom 18 erhalten (25 kg/h), der in Summe 81% T3PN, C3PN und 4PN sowie 3% T2PN, 1% C2PN und 3% E2M2BN enthält. Strom 18 wird als Rückführstrom in Reaktor R1 zurückgefahren.

### Beispiel 2:

In Beispiel 1 wird für die Hydrocyanierung von Butadien ein Katalysatorsystem auf Basis von Nickel(0)-Komplexen mit einem Gemisch von Liganden eingesetzt. Die Ligandmischung zur Hydrocyanierung enthält ca. 80 Mol% Tri(m/p-tolyl)phosphit und 20 Mol% des Chelatphosphits 2: 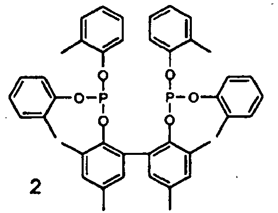 In einem Schlaufenreaktor R1 von 250 l Volumen, der mit einer Strahldüse, Impulsaustauschrohr, externem Umpumpkreislauf und Wärmetauscher zur Abfuhr der Reaktionswärme ausgestattet ist, werden folgende Ströme dosiert:
(1) 12 kg/h flüssiger, unstabilisierter, durch Destillation von Wasser befreiter Cyanwasserstoff,
(2) 39 kg/h Pentennitril, erhalten aus einem Verfahren zu Herstellung von linearem Pentennitril gemäß der DE-A-102 004 004 671, bestehend aus in Summe 98% T3PN, C3PN und 4PN sowie 2% E2M2BN,
(3) 78 kg/h Pentennitril, erhalten als Strom 18 aus der Kolonne K9 aus Schritt (9), enthaltend in Summe 84% T3PN, C3PN, 4PN sowie 2% T2PN, 1% C2PN und 3% E2M2BN.
(4) 5 kg/h Nickel(0)-Katalysatorlösung, bestehend aus 40% Ligandmischung, 2% Nickel(0) und 3% ZnCl₂, hergestellt gemäß Beispiel 4 der DE-A-103 51 002.

Das aus dem Reaktor R1 abgezogene Strom 1 enthält in Summe 54% T3PN, C3PN und 4PN sowie 34% ADN und 3% MGN, entsprechend einem Umsatz von 35% Pentennitril.

Strom 1 wird in einem Schritt 2 in einer Verdampferstufe B2 zugeführt, die mit Fallfilmverdampfer und einem Kopfkondensator ausgestattet ist. Die Verdampferstufe B2 wird bei einem absoluten Druck von 20 mbar Kopfdruck, 313 K Kopftemperatur und 343 K Sumpfabzugstemperatur betrieben.
Über Kopf des Verdampfers B2 wird der Strom 4 erhalten (64 kg/h), der in Summe 82% T3PN, C3PN und 4PN sowie in Summe 5% T2PN und C2PN enthält.

Über Sumpf des Verdampfers B2 werden 65 kg/h eines Stromes 3 mit einem Gehalt von in Summe 19% T3PN, C3PN und 4PN sowie in Summe 1 % T2PN und C2PN erhalten. Strom 3 enthält zudem die Katalysatorkomponenten Nickel(0); den Ligandmischung und ZnCl₂ sowie Katalysatorabbauprodukte.

Strom 3 wird in einem Schritt (3) am unteren Ende einer Gegenstromextraktionskolonne K3 aufgegeben und mit 97 kg/h eines Strom 5, enthaltend 94% Methylcyclohexan sowie verschiedene Pentennitril-Isomere, extrahiert. Der am Kopf der Extraktion erhaltene Strom 6 besteht zu 92% aus dem Extraktionsmittel und enthält neben in Summe 4% T3PN, C3PN und 4PN die Katalysatorkomponenten Nickel(0) und Ligandmischung. Das Zinkchlorid verbleibt vollständig im Sumpfabzug der Extraktionskolonne in Strom 7.

Strom 6 wird in einem Schritt (4) in eine Destillationskolonne K4 gefahren, die mit Fallfilmverdampfer, einem geteilten Sumpf und Kopfkondensator sowie Kolonneneinbauten mit strukturierter Packung ausgestattet ist, die 12 theoretische Trennstufen erzeugen. Die Kolonne wird bei einem absoluten Druck von 125 mbar Kopfdruck, 308 K Kopftemperatur und 361 K Sumpfabzugstemperatur betrieben. Das Rücklaufverhältnis wird so eingestellt, dass in Summe 5% T3PN, C3PN und 4PN im Kopfabzugsstrom enthalten sind.

Zusätzlich zum Strom 6 werden zur Kolonne K4 3 kg/h 3PN dosiert, bestehend aus in Summe 98% T3PN, C3PN und 4PN sowie geringen Mengen anderer Pentennitrile.

Über Kopf der Kolonne K4 wird das Extraktionsmittel zurückgewonnen, das zusammen mit einem kleinen makeup-Strom (10 g/h) von reinem, auf Wasserkonzentration unter 10 ppm getrocknetem Methylcyclohexan zum Ausgleich von Verlusten als Strom 9 in die Extraktionskolonne K3 zurückgefahren wird.

Am Sumpf der Kolonne K4 wird in Strom 8 der extrahierte Katalysator als Lösung in Pentennitril gewonnen (5 kg/h). Strom 8 enthält 38% Ligandmischung, 1% Ni(0) und in Summe 44% T3PN, C3PN und 4PN. Der Methylcyclohexangehalt in Strom 8 wird in der Kolonne K4 auf 10 Gew.-ppm in Strom 8 eingestellt.

Der in der Extraktionskolonne K3 erhaltene Strom 7 wird in einem Schritt (5) zu einer Destillationskolonne K5 gefahren, die mit Zwangsumlaufentspannungsverdampfer, geteiltem Sumpf, Verstärkungs- und Abtriebsteil mit Kolonneneinbauten aus strukturierter Packung ausgestattet ist, die 9 theoretische Trennstufen erzeugen. Die Kolonne K5 wird bei einem absoluten Druck von 460 mbar Kopfdruck, 342 K Kopftemperatur und 433 K Sumpfabzugstemperatur betrieben.

Über Kopf der Kolonne K5 wird ein Strom 11 erhalten, der in die Destillationskolonne K4 gefahren wird. Das Rücklaufverhältnis der Kolonne K5 wird so eingestellt, dass im hauptsächlich aus Methylcyclohexan (87%) bestehenden Kopfprodukt 5% T3PN enthalten sind.

Über Sumpf der Kolonne K5 wird ein Strom 10 erhalten (62 kg/h), der in Summe noch 21% T3PN, C3PN und 4PN sowie 70% ADN und das Nebenprodukt MGN und zusätzlich Katalysatorabbauprodukte zusammen mit ZnCl₂ und Nickelcyaniden enthält, die in der Hydrocyanierung in Reaktor R1 gebildet werden.

Der in der Extraktionskolonne K5 erhaltene Strom 10 wird in einem Schritt (6) zu einer Destillationskolonne K6 gefahren, die mit Zwangsumlaufentspannungsverdampfer, geteiltem Sumpf und einem Verstärkungsteil mit Kolonneneinbauten aus strukturierter Packung ausgestattet ist, die 4 theoretische Trennstufen erzeugen. Die Kolonne K6 wird bei einem absoluten Druck von 15 mbar Kopfdruck, 308 K Kopftemperatur und 426 K Sumpfabzugstemperatur betrieben.

Über Kopf der Kolonne K6 wird ein Strom 13 erhalten, der in die Destillationskolonne K9 von Schritt (9) gefahren wird. Das Rücklaufverhältnis der Kolonne K6 wird so eingestellt, dass im hauptsächlich aus Pentennitrilen (93%) bestehenden Kopfprodukt 100 ppm MGN enthalten sind.

Über Sumpf der Kolonne K6 wird ein Strom 12 erhalten (62 kg/h), der in Summe noch 1000 ppm Pentennitrile, 89% ADN und 11% Nebenprodukt MGN und zusätzlich Katalysatorabbauprodukte, ZnCl₂ und Nickelcyanide enthält.

Strom 12 wird in einem Schritt (7) in einer Verdampferstufe B7 aufgearbeitet, die mit Zwangsumlaufentspannungsverdampfer, Kopfkondensator und nachgeschaltetem Dünnschichtverdampfer B7a zum Einengen des Sumpfproduktes ausgestattet ist. Der Verdampfer wird bei einem absoluten Druck von 1 mbar und 365 K Sumpftemperatur betrieben.

Über Kopf des Verdampfers B7 wird als Roh-ADN der Strom 15 abgezogen (49 kg/h), der neben ADN in Summe 0,1% T3PN, C3PN und 4PN sowie 11% MGN enthält.

Über Sumpf des Dünnschichtverdampfers B7a werden bei 493 K eingeengte Katalysatorrückstände zusammen mit Zinkchlorid als Strom 14 (0,3 kg/h) erhalten, die zur Verflüssigung mit 0,1 kg/h MGN aus dem Kopf der Kolonne K7 aus Schritt (8) versetzt werden.

Strom 15 wird in einem Schritt (8) in eine Destillationskolonne K8 gefahren, die mit Fallfilmverdampfer, Kopfkondensator sowie Kolonneneinbauten mit strukturierter Packung ausgestattet ist, die 35 theoretische Trennstufen erzeugen. Die Kolonne K8 wird bei einem absoluten Druck von 7 mbar Kopfdruck, 393 K Kopftemperatur und 443 K Sumpfabzugstemperatur betrieben. Das Rücklaufverhältnis wird so eingestellt, dass im Kopfprodukt 0,10% ADN enthalten sind. Die Kopfabzugsmenge der Kolonne K8 wird so geregelt, dass im Sumpfprodukt 50 Gew.-ppm MGN enthalten sind.

Der Strom 4 aus dem Verdampfer B2 wird in Schritt (9) in einer Destillationskolonne K9 aufgearbeitet, die mit Umlaufverdampfer, Kopfkondensator sowie Kolonneneinbauten mit strukturierter Packung ausgestattet ist, die 35 theoretische Trennstufen erzeugen. Die Kolonne von Schritt (9) wird bei einem absoluten Druck von 46 mbar Kopfdruck, 313 K Kopftemperatur und 338 K Sumpfabzugstemperatur betrieben. Das Rücklaufverhältnis wird so eingestellt, dass im Kopfprodukt noch 500 ppm T3PN enthalten sind.

Über Kopf der Kolonne K9 wird der Strom 19 erhalten (1,6 kg/h), der 66% C2PN und 29% E2M2BN sowie geringe Mengen VCH und VSN enthält.

Über Sumpf der Kolonne K9 wird der Strom 18 erhalten (78 kg/h), der in Summe 84% T3PN, C3PN und 4PN sowie 2% T2PN, 1 % C2PN und 3% E2M2BN enthält. Strom 18 wird als Rückführstrom in Reaktor R1 zurückgefahren.

## Patentansprüche

1. Verfahren zur Herstellung von Adipodinitril und Methylglutarnitril, **gekennzeichnet durch** die folgenden Verfahrensschritte:
(a) Umsetzung eines Pentennitrile enthaltenden Eduktstroms mit Cyanwasserstoff in Gegenwart mindestens eines Katalysators und mindestens eines Promotors unter Erhalt eines Reaktionsstroms, der Pentennitrile, den mindestens einen Nickel(O)Phosphorligand-Komplex als Katalysator, Katalysatorabbauprodukte, den mindestens einen Promotor aus der Gruppe bestehend aus den Lewissäuren ZnCl₂, FeCl₂, Et₂AlCl, Et₃Al₂Cl₃, EtAlCl₂ und BPh₃, Adipodinitril und Methylglutarnitril, enthält,
(b) Destillation des Reaktionsstroms unter Erhalt eines an Pentennitrilen auf 5 bis 30 Gew.-%abgereicherten Stromes 3, der den mindestens einen Katalysator, Katalysatorabbauprodukte, den mindestens einen Promotor, Adipodinitril und Methylglutarnitril enthält, als Sumpfprodukt und eines an Pentennitrilen angereicherten Stromes 4 als Kopfprodukt,
(c) Extraktion des Stromes 3 mit einem Extraktionsmittel, das ausgewählt ist aus der Gruppe, bestehend aus Cyclohexan, Methylcyclohexan, n-Hexan, n-Heptan, isomeren C6-, C7-, C8-, C9-Cycloaliphaten, isomeren C6-, C7-, C8-, C9-Isoaliphaten, cis-, trans-Decahydronaphthalin und Gemischen davon, enthalten in Strom 5 unter Erhalt eines mit Extraktionsmittel angereicherten Stromes 6 als Kopfprodukt, der den Katalysator enthält, und eines an Extraktionsmittel abgereicherten Stromes 7 als Sumpfprodukt, der Katalysatorabbauprodukte, den mindestens einen Promotor, Pentennitrile, Adipodinitril und Methylglutarnitril enthält,
(d) Destillation des Stromes 6 unter Erhalt eines den Katalysator enthaltenden Stromes 8 als Sumpfprodukt und eines das Extraktionsmittel enthaltenden Stromes 9 als Kopfprodukt, wobei Strom 9 weniger als 10 Gew.-% Pentennitrile enthält und wobei 3-Pentennitril als Zwischensieder zur Destillation gegeben wird,
(e) Destillation des Stromes 7 unter Erhalt eines Stromes 10 als Sumpfprodukt, der Katalysatorabbauprodukte, den mindestens einen Promotor, Pentennitrile, Adipodinitril und Methylglutarnitril enthält, und eines das Extraktionsmittel enthaltenden Stromes 11 als Kopfprodukt,
(f) Destillation des Stromes 10 unter Erhalt eines Stromes 12 als Sumpfprodukt, der Katalysatorabbauprodukte, den mindestens einen Promotor, Adipodinitril und Methylglutarnitril enthält, und eines Pentennitrile enthaltenden Stromes 13 als Kopfprodukt,
wobei der an Pentennitrilen angereicherte Strom 4 und/oder der Strom 13 cis-2-Pentennitril und (E)-2-Methyl-2-butennitril enthält und zumindest teilweise unter Erhalt eines an cis-2-Pentennitril und (E)-2-Methyl-2-buten-nitril abgesicherten Stromes 18 und eines an cis-2-Pentennitril und (E)-2-Methyl-2-butennitril angereicherten Stromes 19 destilliert und der Strom 18 zumindest teilweise in Verfahrensschritt (a) zurückgeführt wird,
(g) Destillation des Stromes 12 unter Erhalt eines Stromes 14 als Sumpfprodukt, der Katalysatorabbauprodukte und den mindestens einen Promotor enthält, und eines Stromes 15 als Kopfprodukt, der Adipodinitril und Methylglutarnitril enthält,
(h) Destillation des Stromes 15 unter Erhalt eines Adipodinitril enthaltenden Stromes 16 als Sumpf und eines Methylglutarnitril enthaltenden Stromes 17 als Kopfprodukt.

2. Verfahren nach Anspruch 1, **dadurch gekennzeichnet, dass** der Eduktstrom aus einer homogenen Hydrocyanierung von Butadien in Gegenwart eines Nickel(0)-Katalysators stammt.

3. Verfahren nach einem der Ansprüche 1 oder 2, **dadurch gekennzeichnet, dass** das verwendete Extraktionsmittel wasserfrei ist.

4. Verfahren nach einem der Ansprüche 1 bis 3, **dadurch gekennzeichnet, dass** der Strom 9 und/oder der Strom 11 zumindest teilweise in den Verfahrensschritt (c) zurückgeführt werden.

5. Verfahren nach einem der Ansprüche 1 bis 4, **dadurch gekennzeichnet, dass** Verfahrensschritt (g) als zweistufige Destillation ausgeführt wird, indem der in Verfahrensschritt (g) erhaltene Strom 14 in einem anschließenden Verfahrensschritt (m) bei einem absoluten Druck von 0,0001 bis 0,5 bar durch Destillation bei einer Temperatur im Sumpf der Destillationsapparatur von 60 bis 350 °C ausgequetscht wird und der ausgequetschte Strom 14 mit mindestens einem Teil des in Verfahrensschritt (h) erhaltenen Stroms 17 enthaltend Methylglutarnitril, verdünnt wird.

## Claims

1. A process for preparing adiponitrile and methylglutaronitrile, **characterized by** the following process steps:
(a) Reacting a reactant stream comprising pentenenitriles with hydrogen cyanide in the presence of at least one catalyst and of at least one promoter to obtain a reaction stream which comprises pentenenitriles, the at least one nickel(0)-phosphorus ligand complex as a catalyst, catalyst degradation products, the at least one promoter from the group consisting of the Lewis acids ZnCl₂, FeCl₂, Et₂AlCl, Et₃Al₂Cl₃, EtAlCl₂ und BPh₃, adiponitrile and methylglutaronitrile,
(b) distilling the reaction stream to obtain a stream 3 which is depleted in pentenenitriles to 5 to 30 % by weight and comprises the at least one catalyst, catalyst degradation products, the at least one promoter, adiponitrile and methylglutaronitrile as the bottom product, and a stream 4 enriched in pentenenitriles as the top product,
(c) extracting the stream 3 using an extractant which is selected from the group consisting of cyclohexane, methylcyclohexane, n-hexane, n-heptane, isomeric C6, C7, C8, C9 cycloaliphatics, isomeric C6, C7, C8, C9 isoaliphatics, cis-, trans-decahydronaphthalene and mixtures thereof present in stream 5 to obtain a stream 6 enriched with extractant as the top product which comprises the catalyst, and a stream 7 depleted in extractant as the bottom product which comprises catalyst degradation products, the at least one promoter, pentenenitriles, adiponitrile and methylglutaronitrile,
(d) distilling the stream 6 to obtain a stream 8 comprising the catalyst as the bottom product and a stream 9 comprising the extractant as the top product, wherein the stream 9 comprises less than 10% by weight of pentenenitriles and wherein 3-pentenenitrile is added tot he distillation as medium boiler,
(e) distilling the stream 7 to obtain a stream 10 as the bottom product which comprises catalyst degradation products, the at least one promoter, pentenenitriles, adiponitrile and methylglutaronitrile, and a stream 11 comprising the extractant as the top product,
(f) distilling the stream 10 to obtain a stream 12 as the bottom product which comprises catalyst degradation products, the at least one promoter, adiponitrile and methylglutaronitrile, and a stream 13 comprising pentenenitriles as the top product,
wherein the stream 13 and/or the stream 4 enriched in pentenenitriles comprises cis-2-pentenenitrile and (E)-2-methyl-2-butenenitrile and is at least partly distilled to obtain a stream 18 depleted in cis-2-pentenenitrile and (E)-2-methyl-2-butenenitrile, and a stream 19 enriched in cis-2-pentenenitrile and (E)-2-methyl-2-butenenitrile, and the stream 18 is recycled at least partly into process step (a),
(g) distilling the stream 12 to obtain a stream 14 as the bottom product which comprises catalyst degradation products and the at least one promoter, and a stream 15 as the top product which comprises adiponitrile and methylglutaronitrile,
(h) distilling the stream 15 to obtain a stream 16 comprising adiponitrile as the bottom and a stream 17 comprising methylglutaronitrile as the top product.

2. The process according to claim 1, wherein the reactant stream stems from a homogeneous hydrocyanation of butadiene in the presence of a nickel(0) catalyst.

3. The process according to either of claims 1 and 2, wherein the extractant used is anhydrous.

4. The process according to any of claims 1 to 3, wherein stream 9 and/or stream 11 are recycled at least partly into process step (c).

5. The process according to any of claims 1 to 4, wherein process step (g) is performed as a two-stage distillation, by squeezing the stream 14 obtained in process step (g) in a subsequent process step (m) at an absolute pressure of from 0.0001 bo 0.5 bar by distillation at a temperature of from 60 to 350 °C in the bottoms of the distillation apparatus, and diluting the squeezed stream 14 with at least a portion of the stream 17 obtained in process step (h) and comprising methylglutaronitrile.

## Revendications

1. Procédé de preparation d'adiponitrile et de méthylglutaronitrile, **caractérisé par** les étapes suivantes:
(a) Réaction d'un courant de réactif contenant des pentènenitriles avec l'acide cyanurique en presence d'au moins un catalyseur et d'au moins un promoteur, avec obtention d'un courant de reaction, qui contient des pentènenitriles, le au moins un complexe nickel(0)-ligand phosphore comme catalyseur, les produits de degradation du catalyseur, le au moins un promoteur choisi parmi le groupe consistant en les acides de Lewis ZnCl₂, FeCl₂, Et₂AlCl, Et₃Al₂Cl₃, EtAlCl₂ et BPh₃, l'adiponitrile et le méthylglutaronitrile,
(b) Distillation du courant de reaction pour obtenir un courant 3 appauvri en pentènenitriles jusqu'à 5 à 30 % en poids, qui contient le au moins un catalyseur, les produits de degradation du catalyseur, le au moins un promoteur, l'adiponitrile et le mèthylglutaronitrile, comme produit de fond, et un courant 4 enrichi en pentènenitriles comme produit de tête,
(c) Extraction du courant 3 avec un agent d'extraction, qui est choisi parmi le groupe consistant en le cyclohexane, le mèthylcyclohexane, le n-hexane, le n-heptane, des cycloaliphatiques en C6, C7, C8, C9 isomères, des isoaliphatiques en C6, C7, C8, C9 isomères, le cis-, le trans-décahydronaphtalène et leurs mélanges, present dans le courant 5, pour obtenir un courant 6 enrichi en agent d'extraction comme produit de tête, qui contient le catalyseur, et us courant 7 appauvri en agent d'extraction, comme produit de fond, qui contient les produits de degradation du catalyseur, le au moins un promoteur, les pentènenitriles, l'adiponitrile et le méthylglutaronitrile,
(d) Distillation du courant 6 pour obtenir un courant 8 contenant le catalyseur comme produit de fond et un courant 9 contenant l'agent d'extraction comme produit de tête, où le courant 9 contient moins de 10% en poids de pentènenitriles et où 3-pentènenitrile est ajouté à la distillation comme ébulliant intermédiaire,
(e) Distillation du courant 7 pour obtenir un courant 10 comme produit de fond, qui contient les produits de degradation du catalyseur, le au moins un promoteur, les pentènenitriles, l'adiponitrile et le méthylglutaronitrile, et un courant 11 contenant l'agent d'extraction, comme produit de tête,
(f) Distillation du courant 10 pour obtenir un courant 12 comme produit de fond, qui contient les produits de degradation du catalyseur, le au moins un promoteur, l'adiponitrile et le méthylglutaronitrile, et un courant 13 contenant les pentènenitriles comme produit de tête, où le courant 4 enrichi en pentènenitriles et/ou le courant 13 contiennent le cis-2-pentènenitrile et le (E)-2-méthyl-2-butènenitrile et est distillé au moins partiellement pour obtenir un courant 18 appauvri en cis-2-pentènenitrile et en (E)-2-méthyl-2-butènenitrile et un courant 19 enrichi en cis-2-pentènenitrile et en (E)-2-méthyl-2-butènenitrile, et le courant 18 est recycle au moins partiellement à l'étape (a),
(g) Distillation du courant 12 pour obtenir un courant 14 comme produit de fond, qui contient les produits de degradation du catalyseur et le au moins un promoteur, et un courant 15 comme produit de tête, qui contient l'adiponitrile et le méthylglutaronitrile,
(h) Distillation du courant 15 pour obtenir un courant 16 contenant l'adiponitrile comme fond et un courant 17 contenant le méthylglutaronitrile comme produit de tête.

2. Procédé selon la revendication 1, **caractérisé en ce que** le courant de réactif provident d'une hydrocyanuration homogène du butadiene en presence d'un catalyseur au nickel (0).

3. Procédé selon la revendication 1 ou 2, **caractérisé en ce que** l'agent d'extraction utilize est anhydre.

4. Procédé selon l'une des revendications 1 à 3, **caractérisé en ce que** le courant 9 et/ou le courant 11 sont recycles au moins partiellement à l'étape (c).

5. Procédé selon l'une des revendications 1 à 4, **caractérisé en ce que** l'étape (g) est réalisée sous forme d'une distillation en deux étapes, dans laquelle le courant 14 obtenu à l'étape (g) est traité dans une étape (m) à une pression absolue de 0.0001 à 0.5 bar par distillation à une temperature de 60 à 350 °C dans le fond de l'appareil de distillation suivante et le courant 14 traité est dilué avec au moins une partie du courant 17 obtenu à l'étape (h) contenant le méthylglutaronitrile.
